# EUROPEAN PATENT APPLICATION

(11) **EP 4 461 328 A1**
(43) Date of publication of application: **13.11.2024**
(21) Application number: 24165877.2
(22) Date of filing: 25.03.2024
(51) Int. Cl.: A61M 5/145

(54) **SYRINGE FIXING DEVICE, SYRINGE PUMP AND CONTROL METHOD THEREFOR**

(30) Priority: 12.05.2023 CN 202310541934; 30.06.2023 CN 202310804933
(71) Applicant: Medcaptain Medical Technology Co., Ltd., Shenzhen, Guangdong 518000 (CN)
(72) Inventor: Chen, Rui, Nanshan, Guangdong, 518000 (CN); Liu, Fulin, Nanshan, Guangdong, 518000 (CN); Huang, Haoke, Nanshan, Guangdong, 518000 (CN); Dong, Jun, Nanshan, Guangdong, 518000 (CN); Deng, Jianzhong, Nanshan, Guangdong, 518000 (CN)
(74) Representative: Grünecker Patent- und Rechtsanwälte PartG mbB

(57) **Abstract**

Embodiments of the present application relates to the technical field of medical apparatuses, and in particular to a syringe fixing device, a syringe pump and a control method therefor, which are intended to solve the problem in the related art of a liquid drug in a syringe being injected into a body uncontrollably. The syringe fixing device comprises a cylindrical body, a fixing block, a brake member, a transmission rod, an elastic compression member and a first driving mechanism. The cylindrical body and a pump body are slidable, and an inner wall of the cylindrical body is provided with a limiting portion; the fixing block is connected to a first end of the cylindrical body, and a first accommodating cavity having an opening is provided in the fixing block; the brake member is located in the first accommodating cavity; the transmission rod is slidably arranged in the cylindrical body, and a first end of the transmission rod is connected to the brake member; a first end of the elastic compression member abuts against the limiting portion, and a second end of the elastic compression member abuts against the brake member; and the first driving mechanism is connected to a second end of the transmission rod. The syringe fixing device can fix a handle of the syringe to prevent the liquid drug in the syringe from being injected into the body uncontrollably.

## Description

### TECHNICAL FIELD

Embodiments of the present application belong to the technical field of medical apparatuses, and in particular, to a syringe fixing device, a syringe pump and a control method therefor.

### BACKGROUND ART

A syringe pump is a medical apparatus for injecting a minute amount of liquid drug in a syringe into a body accurately, uniformly and continuously. The syringe pump generally includes a pump body, a pump door, a syringe fixing device and a pushing device, a mounting space is formed between the pump body and the pump door, and the mounting space is configured to mount the syringe. The syringe fixing device is connected to the pump body, and after the syringe is mounted in the mounting space, the syringe fixing device will fix the syringe so as to accurately position the syringe. Next, the pushing device is engaged with a handle of the syringe, so as to push the handle of the syringe to inject the liquid drug. However, during the fixing and engagement processes of the syringe, the liquid drug in the syringe is injected into the body uncontrollably, causing the injection accuracy of the liquid drug to be reduced.

### SUMMARY OF THE INVENTION

In view of this, embodiments of the present application provide a syringe fixing device, a syringe pump and a control method therefor, in order to solve the technical problem of a liquid drug in a syringe being injected into a body uncontrollably during the fixing and engagement processes of the syringe.

A first aspect of the embodiments of the present application provides a syringe fixing device for fixing a syringe placed in a pump body, the syringe fixing device comprising a cylindrical body, a fixing block, a brake member, a transmission rod, an elastic compression member and a first driving mechanism, wherein the cylindrical body is connected to the pump body and is slidable relative to the pump body, and an inner wall of the cylindrical body is provided with a limiting portion; the fixing block is connected to a first end of the cylindrical body away from the pump body and abuts against a barrel of the syringe, a first accommodating cavity in communication with the cylindrical body is provided in the fixing block, and the first accommodating cavity has an opening facing the syringe; the brake member is located in the first accommodating cavity, and the brake member has a braking portion opposite to the opening; the transmission rod is slidably arranged in the cylindrical body, and a first end of the transmission rod extends into the first accommodating cavity and is connected to the brake member; the elastic compression member is located in the first accommodating cavity and the cylindrical body that are in communication with each other, a first end of the elastic compression member abuts against the limiting portion, and a second end of the elastic compression member abuts against the brake member; and the first driving mechanism is connected to a second end of the transmission rod, for driving the transmission rod to slide relative to the cylindrical body;
wherein when the syringe fixing device is in a first fixing state, the first driving mechanism drives the transmission rod to slide the brake member toward the syringe, the brake member compresses the elastic compression member, the elastic compression member pushes, by means of the limiting portion, the cylindrical body and the fixing block to slide toward the syringe, and the fixing block abuts against the barrel of the syringe; and when the syringe fixing device is in a second fixing state, the first driving mechanism continues to drive the transmission rod to slide the brake member toward the syringe, and the braking portion of the brake member extends from the opening to abut against a handle of the syringe.

According to the syringe fixing device of the embodiments of the present application, the first driving mechanism can be controlled to act after the syringe is placed in the pump body, such that the syringe fixing device is in the first fixing state, that is, the fixing block of the syringe fixing device abuts against the barrel of the syringe, so as to fix the barrel of the syringe. The first driving mechanism continues to be controlled to act such that the syringe fixing device is in the second fixing state, that is, the braking portion of a brake pad of the syringe fixing device abuts against the handle of the syringe to fix the handle of the syringe. Accordingly, in the process of fixing the syringe, in the subsequent process of engaging the handle of the syringe, when there is a negative pressure in the syringe, when the handle of the syringe is touched accidentally, or in other cases, and when an abnormality occurs in the syringe pump, the handle of the syringe can be prevented from moving relative to the barrel, and the liquid drug in the syringe can then be prevented from being injected into the body uncontrollably, so that the liquid drug injection accuracy and the safety performance of the syringe pump are improved.

In some possible implementations, the transmission rod is provided with a transmission thread on an outer side of the transmission rod close to the second end thereof; and the first driving mechanism comprises a driving motor and a transmission nut, the driving motor is mounted on the pump body, an output shaft of the driving motor is connected to the transmission nut so as to drive the transmission nut to rotate, and the transmission nut is sleeved on the transmission thread.

In some possible implementations, the first driving mechanism further comprises a transmission assembly, the transmission assembly being connected to the driving motor and the transmission nut.

In some possible implementations, the transmission nut is connected to the pump body in such a way that the transmission nut is rotatable about an axis of the transmission nut, and transmission teeth are provided on an outer side of the transmission nut; and the transmission assembly comprises at least one transmission gear, the transmission gear being sleeved on the output shaft of the driving motor and meshing with the transmission teeth.

In some possible implementations, the brake member further comprises a connecting portion, a first end of the connecting portion is connected to the first end of the transmission rod, a second end of the connecting portion is connected to the braking portion, and the connecting portion abuts against the fixing block.

In some possible implementations, the connecting portion is of an arc-shaped structure which is bent toward a side away from the pump body.

In some possible implementations, the fixing block comprises a first fixing portion and a second fixing portion which are connected to each other, the first fixing portion is connected to an end of the cylindrical body facing away from the pump body, a first accommodating groove is provided on a side of the first fixing portion facing away from the cylindrical body, and the opening is provided at a bottom of the first accommodating groove; and the second fixing portion is located on the side of the first fixing portion facing away from the cylindrical body, a second accommodating groove is provided on a side of the second fixing portion facing the first fixing portion, and the second accommodating groove and the first accommodating groove enclose the first accommodating cavity.

In some possible implementations, the first fixing portion comprises a first fixing sub-portion and a second fixing sub-portion which are arranged in a radial direction of the cylindrical body and connected to each other; a part of the second fixing sub-portion protrudes from an end surface of the first fixing sub-portion facing the pump body, and the first fixing sub-portion and the part of the second fixing sub-portion protruding from the first fixing sub-portion form a press-connecting groove configured to be engaged with a flange of the barrel; and the opening is provided in the second fixing sub-portion and is located on a side of the press-connecting groove facing away from the pump body.

In some possible implementations, the syringe fixing device further comprises a guide ring, wherein the guide ring is mounted in a connecting through hole of the pump body, and a guide groove is provided on an inner side of the guide ring; and the cylindrical body is inserted into the guide ring, a guide protrusion is provided on an outer side of the cylindrical body close to a second end thereof, and the guide protrusion is slidably arranged in the guide groove.

In some possible implementations, the guide groove comprises a linear groove section and a spiral groove section which are connected to each other, an extension direction of the linear groove section is parallel to an axis of the guide ring, and the linear groove section has a guide inlet formed at the second end of the cylindrical body; and the spiral groove section is closer to the fixing block than the linear groove section, and an extension direction of the spiral groove section surrounds the axis of the guide ring.

In some possible implementations, a mounting groove is provided in a side surface of the cylindrical body close to the second end thereof, and the mounting groove has a mounting opening formed at the second end of the cylindrical body; the second end of the cylindrical body is provided with a connecting cylinder, and the connecting cylinder has a smaller radius than the cylindrical body; the syringe fixing device further comprises a first mounting frame, the first mounting frame comprises a first mounting ring and a first connecting arm, and the first mounting ring is sleeved on the connecting cylinder; the first connecting arm is arranged in an axial direction of the first mounting ring and is connected to the first mounting ring, and the first connecting arm is mounted in the mounting groove; and the guide protrusion is provided on a side of the first connecting arm facing away from the mounting groove.

In some possible implementations, the syringe fixing device furthercomprises a size sensor, wherein the size sensor is connected to the cylindrical body and is close to the second end of the cylindrical body, and the size sensor is configured to identify the size of the syringe.

In some possible implementations, the syringe fixing device further comprises a second mounting frame, wherein the second mounting frame comprises a second mounting ring and a second connecting arm, and the second mounting ring is sleeved on the connecting cylinder and abuts against the first mounting ring; and the second connecting arm is arranged in an axial direction of the second mounting ring, a first end of the second connecting arm is connected to the second mounting ring, and a second end of the second connecting arm is configured to mount the size sensor.

In some possible implementations, an engagement groove is provided on an outer side of the connecting cylinder, and the engagement groove is located on a side of the second mounting ring facing away from the first mounting ring; and the syringe fixing device further comprises a circlip engaged in the engagement groove.

In some possible implementations, a wave washer is provided between the first mounting ring and the second mounting ring, and the wave washer is sleeved on the connecting cylinder and abuts against each of the first mounting ring and the second mounting ring.

In some possible implementations, the cylindrical body comprises a first cylindrical body and a second cylindrical body, and the first cylindrical body is slidable relative to the pump body; a first end of the second cylindrical body is sleeved on a first end of the first cylindrical body away from the pump body, and is fixedly connected to the first cylindrical body for the transmission rod to pass through; and the first end of the first cylindrical body is the limiting portion, and a second end of the second cylindrical body is connected to the fixing block.

A second aspect of the embodiments of the present application provides a syringe pump, comprising a pump body, a pushing device, and a syringe fixing device as described in any one of the above embodiments, wherein the pump body has a second accommodating space for placing a syringe; and the pushing device is connected to the pump body, and is configured to be engaged with a handle of the syringe so as to drive the handle to slide relative to a barrel of the syringe.

Since the syringe pump in the embodiments of the present application comprises the syringe fixing device as described in any one of the above embodiments, the syringe pump also has the advantages of the syringe fixing device as described in any one of the above embodiments, which will not be described in details herein.

A third aspect of the embodiments of the present application provides a method for controlling a syringe pump. The method is applied to the syringe pump as described above. The method comprises:
generating a first control signal, and transmitting the first control signal to the first driving mechanism of the syringe fixing device, wherein the first control signal instructs to mount a syringe in place;
generating a second control signal, and transmitting the second control signal to the pushing device, wherein the second control signal instructs the pushing device to be engaged with a handle of the syringe; and
generating a third control signal, and transmitting the third control signal to the first driving mechanism, wherein the third control signal instructs the braking portion to move away from the handle of the syringe.

With the method for controlling a syringe pump of the embodiments of the present application, the first driving mechanism can be controlled to act after the syringe is placed in the pump body, such that the syringe fixing device is in the first state, that is, the fixing block of the syringe fixing device abuts against the barrel of the syringe, so as to fix the barrel of the syringe. The first driving mechanism continues to be controlled to act such that the syringe fixing device is in the second state, that is, the braking portion of the brake pad of the syringe fixing device abuts against the handle of the syringe to fix the handle of the syringe. Accordingly, in the process of fixing the syringe, in the subsequent process of engaging the handle of the syringe, when the handle of the syringe is touched accidentally, or in other cases, the handle of the syringe can be prevented from moving relative to the barrel, and the liquid drug in the syringe can then be prevented from being injected into the body uncontrollably, so that the liquid drug injection accuracy and the safety performance of the syringe pump are improved.

In some possible implementations, the syringe pump further comprises an in-place sensor; and the step of generating the first control signal comprises:
receiving a first operation on a first target key; and generating the first control signal in response to the first operation;
or receiving an in-place signal generated by the in-place sensor, and generating the first control signal according to the in-place signal.

In some possible implementations, the pushing device comprises a pushing portion engaged with the syringe; and before the step of generating the first control signal, the method further comprises:
starting the syringe pump, and generating a first opening signal;
or starting the syringe pump, receiving a second operation on a second target key, and generating a first opening signal in response to the second operation; wherein the first opening signal controls the pushing portion to move away from the pump body.

### BRIEF DESCRIPTION OF THE DRAWINGS

In order to more clearly describe the technical solutions in the embodiments of the present application or in the prior art, the accompanying drawings required for describing the embodiments or the prior art will be briefly described below. Apparently, the drawings in the following description merely show some of the embodiments of the present application, and those of ordinary skill in the art would have obtained other drawings according to these drawings without involving any inventive effort.
FIG. 1 is a schematic structural perspective view of a syringe pump according to a first embodiment of the present application from a first perspective;
FIG. 2 is a schematic view of an overall structure of the syringe pump in FIG. 1 with a pump door of the syringe pump being opened;
FIG. 3 is a schematic structural perspective view of a syringe fixing device (with a second housing and a door body hidden) in FIG. 2 in an opened plate;
FIG. 4 is a schematic structural diagram of the syringe fixing device in FIG. 2 in a first fixing state;
FIG. 5 is a schematic structural diagram of the syringe fixing device in FIG. 2 in the first fixing state with a syringe being placed;
FIG. 6 is a schematic structural diagram of the syringe fixing device in FIG. 2 in a second fixing state;
FIG. 7 is a schematic structural perspective view of the syringe fixing device in FIG. 2 from a second perspective;
FIG. 8 is a schematic partial exploded view of the syringe fixing device in FIG. 7;
FIG. 9 is a schematic structural perspective view of the syringe fixing device in FIG. 2 from a third perspective;
FIG. 10 is a schematic structural exploded view of the syringe fixing device (with a first driving mechanism hidden) in FIG. 2;
FIG. 11 is a schematic structural cross-sectional view of the syringe fixing device in FIG. 10;
FIG. 12 is a schematic structural front view of the syringe fixing device (with a first driving mechanism hidden);
FIG. 13 is a schematic structural diagram of the syringe fixing device in FIG. 12 in the first fixing state;
FIG. 14 is a schematic structural cross-sectional view of the syringe fixing device in FIG. 13;
FIG. 15 is a schematic structural diagram of the syringe fixing device in FIG. 12 in the second fixing state;
FIG. 16 is a schematic structural cross-sectional view of the syringe fixing device in FIG. 15;
FIG. 17 is a schematic structural diagram of the first housing in FIG. 2;
FIG. 18 is a schematic structural partial cross-sectional view of the first housing in FIG. 17;
FIG. 19 is a schematic structural diagram of the first housing (with a bottom plate hidden) in FIG. 17 from a fourth perspective;
FIG. 20 is a schematic partial enlarged view of part A in FIG. 19;
FIG. 21 is a schematic structural diagram of a guide ring in FIG. 20 from a fifth perspective;
FIG. 22 is a schematic structural diagram of the guide ring in FIG. 20 from a sixth perspective;
FIG. 23 is a schematic structural diagram of a pushing portion in FIG. 2 from a seventh perspective;
FIG. 24 is a schematic flow chart of a method for controlling a syringe pump according to a second embodiment of the present application;

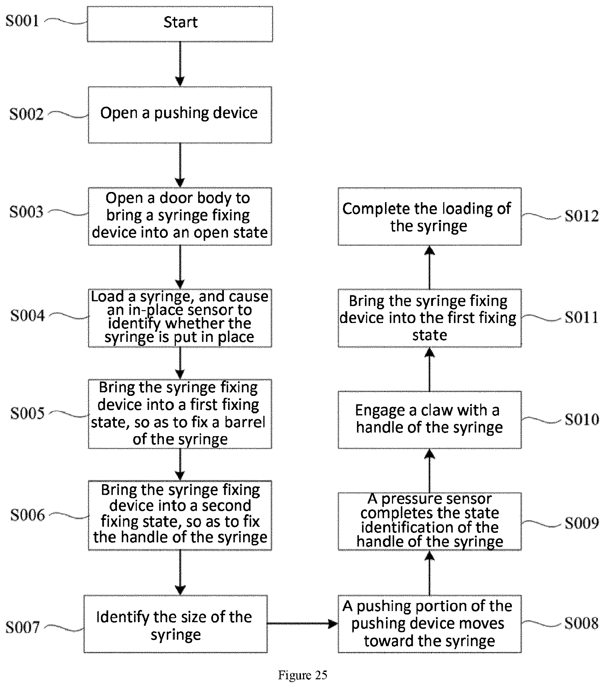

FIG. 25 is a schematic flow chart of a method for controlling a syringe pump according to a third embodiment of the present application;
FIG. 26 is a schematic flow chart of a method for controlling a syringe pump according to a fourth embodiment of the present application;
FIG. 27 is a schematic flow chart of a method for controlling a syringe pump in some possible implementations of a fifth embodiment of the present application;
FIG. 28 is a schematic flow chart of a control method for the syringe pump in some other possible implementations of the fifth embodiment of the present application;
FIG. 29 is a schematic flow chart of a method for controlling a syringe pump according to a sixth embodiment of the present application; and
FIG. 30 is a schematic flow chart of a method for controlling a syringe pump according to a seventh embodiment of the present application.

### List of reference signs:

| | | | |
|---|---|---|---|
| 10 | - Pump body; | | |
| 110 | - First housing; | 111 | - Second accommodating cavity; |
| 112 | - Third accommodating cavity; | 113 | - Fourth accommodating cavity; |
| 114 | - Partition portion; | 115 | - Rotary connecting portion; |
| 116 | - First rotating through hole; | 117 | - Connecting through hole; |
| 118 | - Sliding through hole; | 120 | - Second housing; |
| 121 | - First key; | 130 | - Door body; |
| 131 | - Display screen; | 132 | - Second key; |
| 20 | - Syringe fixing device; | | |
| 210 | - Cylindrical body; | 201 | - Limiting portion; |
| 211 | - First cylindrical body; | 212 | - Second cylindrical body; |
| 213 | - Guide protrusion; | 214 | - Mounting groove; |
| 215 | - Connecting cylinder; | 216 | - First mounting ring; |
| 217 | - First connecting arm; | 218 | - Engagement groove; |
| 219 | - Circlip; | 220 | - Fixing block; |
| 221 | - First accommodating cavity; | 222 | - Opening; |
| 223 | - First fixing portion; | 224 | - Second fixing portion; |
| 225 | - First accommodating groove; | 226 | - First fixing sub-portion; |
| 227 | - Second fixing sub-portion; | 228 | - Press-connecting groove; |
| 229 | - Second accommodating groove; | 230 | - Brake member; |

| | | | |
|---|---|---|---|
| 231 | - Braking portion; | 232 | - Connecting portion; |
| 240 | - Transmission rod; | 241 | - Seal ring; |
| 250 | - Elastic compression member; | 260 | - First driving mechanism; |
| 261 | - Driving motor; | 262 | - Transmission nut; |
| 263 | - Mounting seat; | 264 | - Transmission teeth; |
| 265 | - Transmission gear; | 270 | - Guide ring; |
| 271 | - Guide groove; | 272 | - Linear groove section; |
| 273 | - Spiral groove section; | 280 | - Second mounting frame; |
| 281 | - Second mounting ring; | 282 | - Second connecting arm; |
| 283 | - Wave washer; | 290 | - Clip; |
| 30 | - Pushing device; | | |
| 310 | - Push rod; | 320 | - Pushing portion; |
| 321 | - Pressure sensor; | 322 | - Claw; |
| 330 | - Second driving mechanism; | | |
| 40 | - Syringe; | | |
| 410 | - Barrel; | 420 | - Handle. |

### DETAILED DESCRIPTION OF EMBODIMENTS

In view of the technical problem in the related art of a liquid drug in a syringe being injected into a body uncontrollably during the fixing and engagement processes of the syringe, a syringe fixing device in the embodiments of the present application is provided with a fixing block, a brake member and a first driving mechanism. The fixing block is provided with a first accommodating cavity having an opening, the brake member is located in the first accommodating cavity, the brake member has a braking portion, and the braking portion can extend out through the opening. The first driving mechanism is connected to the fixing block and the brake member. When the syringe fixing device is in a first fixing state, the first driving mechanism drives the fixing block to abut against a barrel of a syringe so as to fix the barrel. When the syringe fixing device is in a second fixing state, the first driving mechanism continues to drive the brake member to move toward the syringe relative to the fixing block, such that the braking portion of the brake member extends from the opening to abut against a handle of the syringe, so as to brake the handle of the syringe to prevent the movement of the handle during the fixing and engagement processes of the syringe, when there is negative pressure in the syringe, when the handle of the syringe is accidentally touched, or in other cases. Moreover, when an abnormality occurs in the syringe pump, the handle is fixed, and the liquid drug in the syringe is thus prevented from being injected into the body uncontrollably, so that the accuracy of injecting the liquid drug is ensured.

In order to make the objectives, technical solutions and advantages of embodiments of the present application clearer, the technical solutions in the embodiments of the present application will be described clearly and completely below with reference to the accompanying drawings in the embodiments of the present application. Apparently, the embodiments described are some of, rather than all of, the embodiments of the present application. All the other embodiments obtained by those of ordinary skill in the art based on the embodiments of the present application without creative efforts shall fall within the scope of protection of the present application.

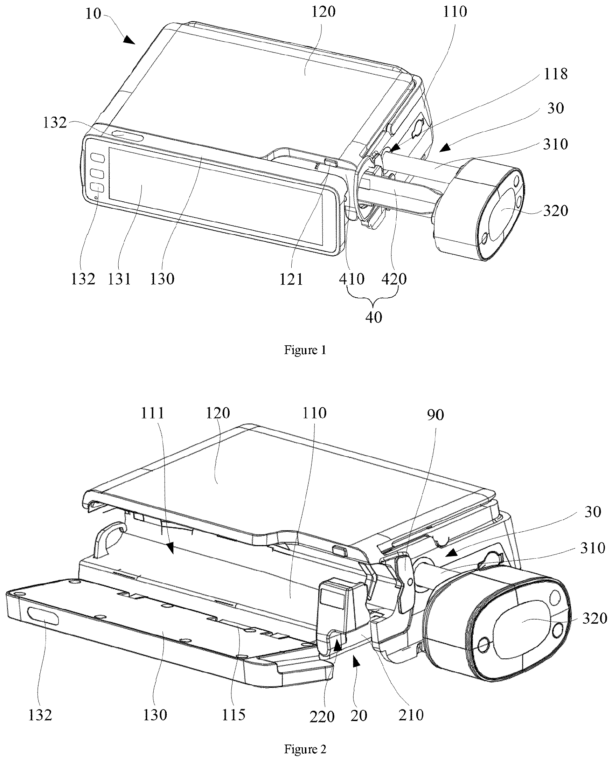

Referring to FIGS. 1 and 2, a first embodiment of the present application provides a syringe pump, which includes a pump body 10, a syringe fixing device 20, and a pushing device 30. The syringe fixing device 20 and the pushing device 30 are both connected to the pump body 10. The syringe fixing device 20 is configured to fix a syringe mounted in the pump body 10. The pushing device 30 is configured to be engaged with a handle of the syringe, and to push the handle to move relative to a barrel of the syringe, so as to inject a liquid drug in the syringe into a body.

As an example, referring to FIGS. 1, 2, 3 and 4, the pump body 10 may include oppositely arranged first housing 110 and second housing 120, and a door body 130. The first housing 110 and the second housing 120 are arranged oppositely and connected to each other. A bottom plate may be connected to a side of the first housing 110 facing away from the second housing 120. The first housing 110 and the second housing 120 may enclose a second accommodating cavity 111, and the second accommodating cavity 111 may be configured to place the syringe.

As an example, an in-place sensor may be provided in the second accommodating cavity 111, and the in-place sensor may be configured to detect whether the syringe is mounted in place. If the syringe 40 is put in place, the in-place sensor emits an in-place signal. If the syringe 40 is not put in place, the in-place sensor emits no in-place signal, or emits a not-in-place signal. As an example, the in-place sensor may be a switch. When the switch is turned on, it is indicated that the syringe is put in place, and when the switch is turned off, it is indicated that the syringe is not put in place. As an example, the switch may be an optocoupler switch, a touch switch, etc.

In some possible implementations of the first embodiment of the present application, the second housing 120 may further be provided with a first key 121. As an example, the first key 121 may be a push key or a rotary key. As an example, after the syringe 40 is placed in the second accommodating cavity 111, an operator may, when confirming that the syringe 40 is put in place, operate the first key 121 to confirm that the syringe 40 has been put in place. For example, the operator may press the first key 121 or rotate the first key 122. It should be understood that the operator may also, by operating the first key 121, trigger actions other than confirming that the syringe 40 is mounted in place, which will not be described in details in the first embodiment of the present application.

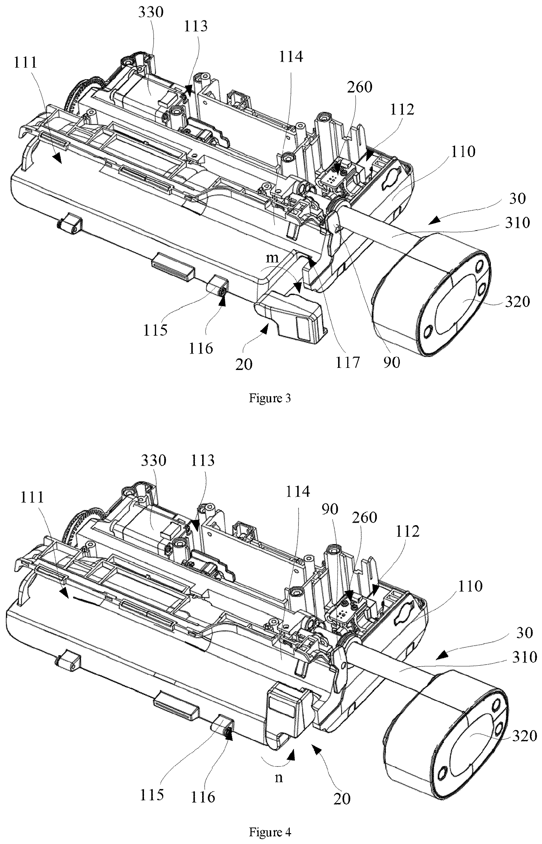

Referring to FIGS. 3 and 4, the first housing 110 and the second housing 120 may also enclose a third accommodating cavity 112. The third accommodating cavity 112 is arranged spaced apart from the second accommodating cavity 111, and the third accommodating cavity 112 is configured to accommodate part of the syringe fixing device 20. For example, the third accommodating cavity 112 may accommodate a first driving mechanism 260 of the syringe fixing device 20.

As an example, the first housing 110 and the second housing 120 may also enclose a fourth accommodating cavity 113, and the fourth accommodating cavity 113 is arranged spaced apart from the second accommodating cavity 111. As an example, the pump body 10 further includes a partition portion 114, the second accommodating cavity 111 is located on one side of the partition portion 114, and the third accommodating cavity 112 and the fourth accommodating cavity 113 are located on the other side of the partition portion 114. The fourth accommodating cavity 113 is configured to accommodate part of the pushing device 30. For example, the fourth accommodating cavity 113 may accommodate a second driving mechanism 330 of the pushing device 30.

The first housing 110 is further connected to the door body 130, and the door body 130 is configured to close or open the second accommodating cavity 111. As an example, one side of the door body 130 may be rotatably connected to the first housing 110, and the second accommodating cavity 111 may be closed or opened by rotating the door body 130. For example, referring to FIGS. 3 and 4, the first housing 110 may be provided with a rotary connecting portion 115. The rotary connecting portion 115 is provided with a first rotating through hole 116. A second rotating through hole is provided in one side of the door body 130, and the second rotating through hole is opposite to the first rotating through hole 116. A rotating shaft may pass through the first rotating through hole 116 and the second rotating through hole that are opposite to each other. The first housing 110 and the door body 130 are rotatably connected to each other by means of the rotating shaft.

As an example, the syringe fixing device 20 may further include a door state sensor. The door state sensor may be mounted on the first housing 110 or the door body 130, or may be mounted on the second housing 120, and the door state sensor is configured to detect the state of the door body 130. When the door body 130 is in a closed state, the door state sensor sends a door closed signal. When the door body 130 is in an open state, the door state sensor does not send the closed signal, or sends a door opened signal. As an example, the door state sensor may be a proximity switch, an infrared transmitter and receiver, etc.

As an example, a display screen 131 may further be mounted on the door body 130, the display screen 131 is configured to display relevant information of the syringe pump, such as the usage state of the syringe pump, and the type, flow rate and injection duration of the injected liquid drug. The display screen 131 may further be configured to display other information, which will not be described in details in the first embodiment of the present application. As an example, the display screen 131 may have a touch function, and the syringe pump may be controlled by touching the display screen 131.

At least one second key 132 may further be provided on the door body 130. The second key 132 may be mounted on a side of the door body 130 that is provided with the display screen 131, or may be mounted on a side surface of the door body 130. As an example, the second key 132 may be a push key or a rotary key. As an example, the operator may operate the second key 132, for example, may press the second key 132, or rotate the second key 132 to open the door body 130. It should be understood that the operator may also, by operating the second key 132, trigger actions other than opening the door body 130, which will not be described in details in the first embodiment of the present application.

As an example, the pump body 10 may further include an alarm light which emits light to provide an alarm when the syringe pump is in an abnormal state, etc.

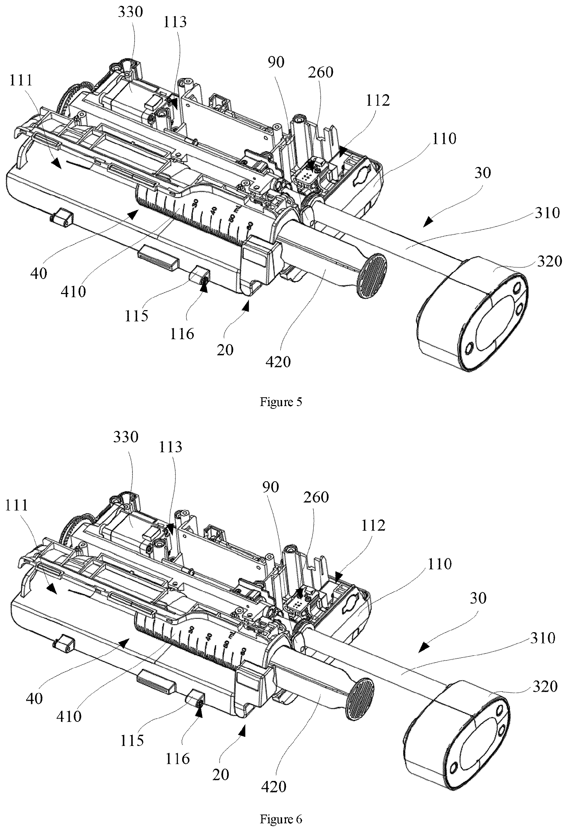

The syringe fixing device 20 is connected to the pump body 10, and is configured to fix the syringe placed in the pump body 10. The syringe fixing device 20 has an open state, a first fixing state and a second fixing state. Referring to FIG. 3, when the syringe fixing device 20 is in the open state, part of the syringe fixing device 20 is away from the pump body 10 so as to facilitate the placement of the syringe in the pump body 10. Referring to FIGS. 4 and 5, when the syringe fixing device 30 is in the first fixing state, the syringe fixing device 20 fixes the barrel 410 of the syringe 40 so as to prevent the movement of the barrel 410. Referring to FIG. 6, when the syringe fixing device 30 is in the second fixing state, the syringe fixing device 20 fixes the handle 420 of the syringe 40, so as to prevent the phenomenon that in the process of fixing the syringe 40, in the subsequent process of engaging the handle 420 of the syringe 40, when there is a negative pressure in the syringe 40, when the handle 420 of the syringe 40 is touched accidentally, or in other cases, the handle 420 of the syringe 40 can be prevented from moving relative to the barrel 410, and the liquid drug in the syringe 40 can then be prevented from being injected into the body uncontrollably, so that the liquid drug injection accuracy and the safety performance of the syringe pump are improved.

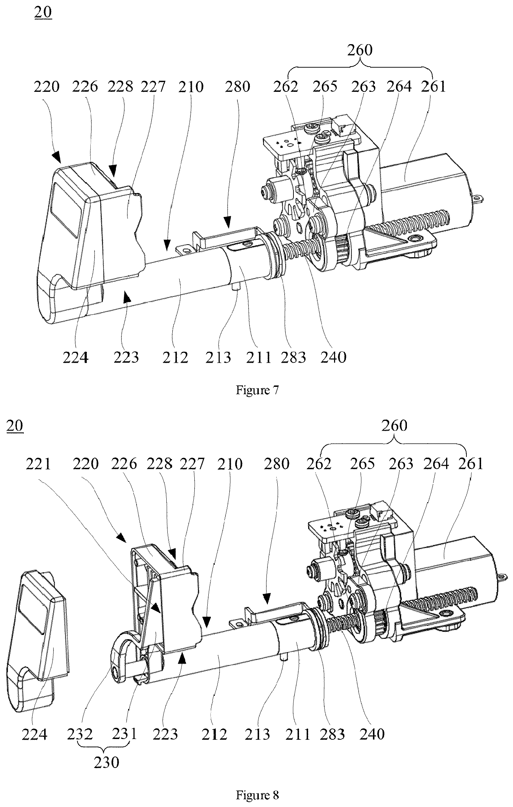

Referring to FIGS. 7, 8, 9, 10, 11 and 12, the syringe fixing device 20 is configured to fix the syringe 40 placed in the pump body 10, and may include a cylindrical body 210, a fixing block 220, a brake member 230, a transmission rod 240, an elastic compression member 250 and a first driving mechanism 260. The cylindrical body 210 is connected to the pump body 10 and can slide relative to the pump body 10, and an inner wall of the cylindrical body 210 is provided with a limiting portion 201. The fixing block 220 is connected to a first end of the cylindrical body 210 away from the pump body 10 and is configured to abut against the barrel 410 of the syringe 40, a first accommodating cavity 221 in communication with the cylindrical body 210 is provided in the fixing block 220, and the first accommodating cavity 221 has an opening 222 facing the syringe 40. The brake member 230 is located in the first accommodating cavity 221, and the brake member 230 has a braking portion 231 opposite to the opening 222. The transmission rod 240 is slidably arranged in the cylindrical body 210, and a first end of the transmission rod 240 extends into the first accommodating cavity 221 and is connected to the brake member 230. The elastic compression member 250 is located in the first accommodating cavity 221 and the cylindrical body 210 that are in communication with each other, a first end of the elastic compression member 250 abuts against the limiting portion 201, and a second end of the elastic compression member 250 abuts against the brake member 230. The first driving mechanism 260 is connected to a second end of the transmission rod 240, for driving the transmission rod 240 to slide relative to the cylindrical body 210.

As an example, after the syringe pump is started, the syringe fixing device 20 may be in the open state to facilitate the placement of the syringe 40. When the syringe fixing device 20 is in the open state, the first driving mechanism 260 drives the transmission rod 240 to slide relative to the cylindrical body 210 toward a side away from the pump body 10, and the transmission rod 240 drives the brake member 230 to slide relative to the cylindrical body 210 toward the side away from the pump body 10. The brake member 230 abuts against the fixing block 220 to exert a thrust on the fixing block 220, such that the fixing block 220 and the cylindrical body 210 connected to the fixing block 220 slide relative to the pump body 10 toward the side away from the pump body 10, so as to move the fixing block 220 away from the first accommodating cavity 221 to facilitate the placement of the syringe 40 in the first accommodating cavity 221.

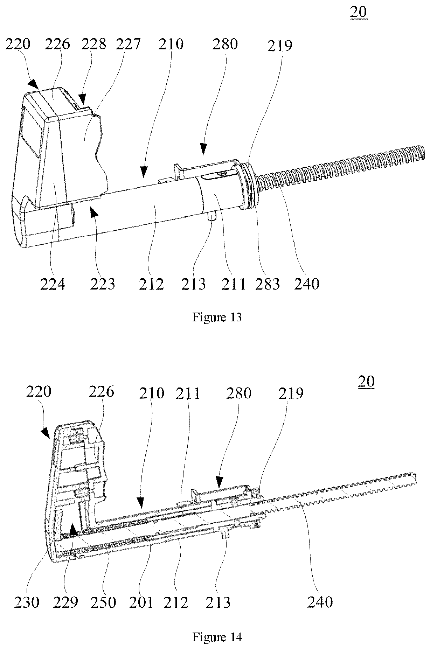

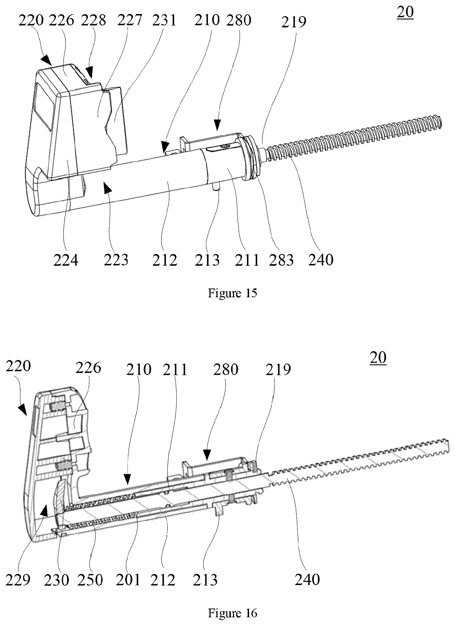

Referring to FIGS. 13 and 14, after the syringe 40 is placed in the pump body 10, the syringe fixing device 20 may be in the first fixing state or the second fixing state. When the syringe fixing device 20 is in the first fixing state, the first driving mechanism 260 drives the transmission rod 240 to slide the brake member 230 toward the syringe 40, the brake member 230 compresses the elastic compression member 250, the elastic compression member 250 pushes, by means of the limiting portion 201, the cylindrical body 210 and the fixing block 220 to slide toward the syringe 40, and the fixing block 220 abuts against the barrel 410 of the syringe 40. Referring to FIGS. 15 and 16, when the syringe fixing device 20 is in the second fixing state, the first driving mechanism 260 continues to drive the transmission rod 240 to slide the brake member 230 toward the syringe 40, and the braking portion 231 of the brake member 230 extends from the opening 222 to abut against the handle 420 of the syringe 40.

According to the syringe fixing device 20 in the first embodiment of the present application, after the syringe 40 is placed in the pump body 10, the first driving mechanism 260 can be controlled to act such that the syringe fixing device 20 is in the first fixing state, that is, the fixing block 220 of the syringe fixing device 20 abuts against the barrel 410 of the syringe 40, so as to fix the barrel 410 of the syringe 40. The first driving mechanism 260 continues to be controlled to act such that the syringe fixing device 20 is in the second fixing state, that is, the braking portion 231 of the brake member 230 abuts against the handle 420 of the syringe 40 to fix the handle 420 of the syringe 40. Accordingly, in the process of fixing the syringe 40, in the subsequent process of engaging the handle 420 of the syringe 40, when there is a negative pressure in the syringe 40, when the handle 420 of the syringe 40 is touched accidentally, or in other cases, the handle 420 of the syringe 40 can be prevented from moving relative to the barrel 410, the handle 420 can be fixed when an abnormality occurs in the syringe pump, and the liquid drug in the syringe 40 can then be prevented from being injected into the body uncontrollably, so that the liquid drug injection efficiency and the safety performance of the syringe pump are improved.

In some possible implementations of the first embodiment of the present application, the cylindrical body 210 may be of a split structure. As an example, referring to FIGS. 7 to 16, the cylindrical body 210 may include a first cylindrical body 211 and a second cylindrical body 212, and the first cylindrical body 211 can slide relative to the pump body 10. A first end of the second cylindrical body 212 is sleeved on a first end of the first cylindrical body 211 away from the pump body 10, and is fixedly connected to the first cylindrical body 211 for the transmission rod 240 to pass through. The first end of the first cylindrical body 211 is the limiting portion 201, and a second end of the second cylindrical body 212 is connected to the fixing block 220. Since one end of the first cylindrical body 211 extends into the second cylindrical body 212 to directly form the limiting portion 201, there is no need to manufacture the limiting portion 201 in the second cylindrical body 212, so that the difficulty of manufacturing the limiting portion 201 in the cylindrical body 210 is reduced and the difficulty of machining the cylindrical body 210 is thus reduced.

In other possible implementations of the first embodiment of the present application, the cylindrical body 210 may alternatively be of an integral structure. As an example, the cylindrical body 210 may include a third cylindrical body, the third cylindrical body can slide relative to the pump body 10, and an end of the third cylindrical body away from the pump body 10 is connected to the fixing block 220. A protruding portion protruding toward a center line of the third cylindrical body is formed in the third cylindrical body, and the protruding portion is the limiting portion 201. The cylindrical body 210 of the integral structure can allow the number of components in the syringe fixing device 20 to decrease, so that the convenience of mounting and adjusting the syringe fixing device 20 is improved.

The end of the cylindrical body 210 away from the pump body 10 is connected to the fixing block 220. As an example, the fixing block 220 may include a first fixing portion 223 and a second fixing portion 224 which are connected to each other. The first fixing portion 223 is connected to the end of the cylindrical body 210 away from the pump body 10. For example, the first fixing portion 223 may be connected to the second end of the second cylindrical body 212. A first accommodating groove 225 is provided on a side of the first fixing portion 223 facing away from the cylindrical body 210, and an opening 222 is provided at the bottom of the first accommodating groove 225. The second fixing portion 224 is located on the side of the first fixing portion 223 facing away from the cylindrical body 210, a second accommodating groove 229 is provided on a side of the second fixing portion 224 facing the first fixing portion 223, and the second accommodating groove 229 and the first accommodating groove 225 enclose the first accommodating cavity 221.

In the process of assembling the syringe fixing device 20, the brake member 230, the transmission rod 240 and the elastic compression member 250 may first be mounted in the cylindrical body 210 and the first accommodating groove 225, the second accommodating groove 229 of the second fixing portion 224 is then opposite to the first accommodating groove 225 to form the accommodating cavity 221, and the second fixing portion 224 and the first fixing portion 223 are then connected to each other by means of a connecting bolt, etc. By configuring the fixing block 220 to include the first fixing portion 223 and the second fixing portion 224 that are connected to each other, that is, by configuring the fixing block 220 to be of the split structure, the convenience of assembling other components in the syringe fixing device 20 can be improved.

As an example, the first fixing portion 223 and the cylindrical body 210 may be of an integral structure. For example, the first fixing portion 223 and the second cylindrical body 212 may be of an integral structure. With such a configuration, the connection strength between the first fixing portion 223 and the cylindrical body 210 can be increased, the first fixing portion 223 and the cylindrical body 210 can be prevented from being separated from each other, and the structural reliability of the syringe fixing device 20 can be improved.

As an example, the first fixing portion 223 may include a first fixing sub-portion 226 and a second fixing sub-portion 227 which are arranged in a radial direction of the cylindrical body 210 and connected to each other. A part of the second fixing sub-portion 227 protrudes from an end surface of the first fixing sub-portion 226 facing the pump body 10, and the first fixing sub-portion 226 and the part of the second fixing sub-portion 227 protruding from the first fixing sub-portion 226 form a press-connecting groove 228. The press-connecting groove 228 is configured to be engaged with a flange of the barrel 410. When the fixing portion 226 abuts against the barrel 410, the press-connecting groove 228 can be engaged with the flange of the barrel 410, such that the first fixing sub-portion 226 abuts against an outer surface of the barrel 410, and the second fixing sub-portion 227 abuts against the flange of the barrel 410, thereby increasing the contact area between the fixing portion 226 and the barrel 410 and thus improving the fixing performance of the fixing portion 226 on the syringe 40. The opening 222 is provided in the second fixing sub-portion 227 and is located on a side of the press-connecting groove 228 facing away from the pump body 10. When the press-connecting groove 228 is engaged with the flange of the barrel 410, the opening 222 is opposite to the handle 420 of the syringe 40, such that the braking portion 231 of the brake member 230 can extend from the opening 222 and then abut against the handle 420.

The brake member 230 is located in the first accommodating cavity 221. When the syringe fixing device 20 is in the open state and the first fixing state, the brake member 230 is received in the first accommodating cavity 221 and abuts against the fixing block 220. When the syringe fixing device 20 is in the second fixing state, the brake member 230 moves relative to the fixing block 220 and the cylindrical body 210, such that the braking portion 231 of the brake member 230 extends from the opening 222 to abut against the handle 420.

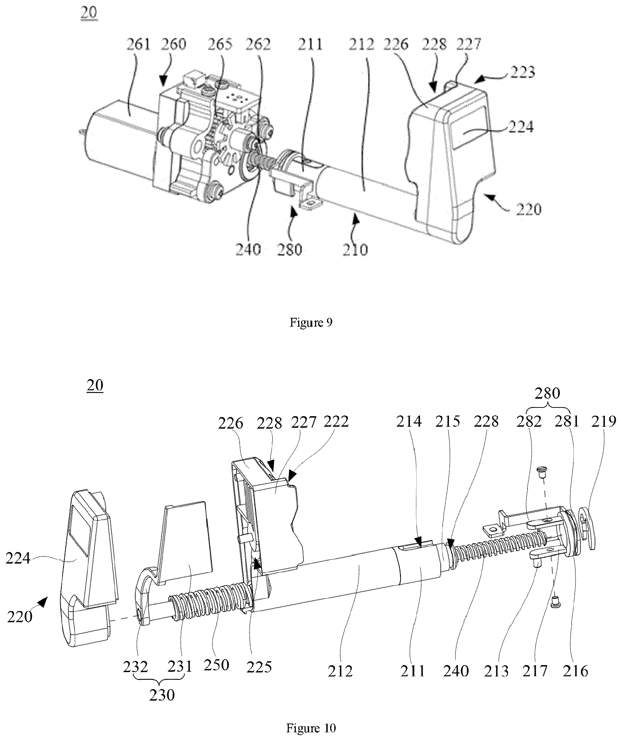

As an example, referring to FIGS. 8 and 10, the brake member 230 may further include a connecting portion 232. A first end of the connecting portion 232 is connected to the first end of the transmission rod 240, a second end of the connecting portion 232 is connected to the braking portion 231, and the connecting portion 232 is configured to abut against the fixing block 220. By providing the connecting portion 232, the contact area between the brake member 230 and the fixing block 220 can be increased. In the process of switching the syringe fixing device 20 into the open state, the brake member 230 can exert a large thrust on the fixing block 220, such that the fixing block 220 can follow the brake member 230 to move away from the pump body 10, and the fixing block 220 can be prevented from blocking the syringe 40.

As an example, the connecting portion 232 may be of an arc-shaped structure which is bent toward a side away from the pump body 10. By configuring the connecting portion 232 to be of the arc-shaped structure, when the fixing block 220 provides a limited accommodating space, the contact area between the brake member 230 and the fixing block 220 can further be increased, and thus the thrust that can be exerted by the brake member 230 on the fixing block 220 can further be increased.

As an example, the connecting portion 232 and the braking portion 231 may be of an integral structure. For example, the connecting portion 232 and the braking portion 231 may be integrally formed by means of casting. The connecting portion 232 and the braking portion 231 that are of the integral structure have a higher connection strength, and the connecting portion 232 and the braking portion 231 can be prevented from being separated from each other, so that the structural reliability of the brake member 230 is improved.

The transmission rod 240 is slidably arranged in the cylindrical body 210, and a first end of the transmission rod 240 extends into the first accommodating cavity 221 and is connected to the brake member 230. As an example, a seal ring 241 may be sleeved on an outer side of the transmission rod 240, the seal ring 241 abuts against an inner wall of the first cylindrical body 211, and the transmission rod 240 may be slidably connected to the first cylindrical body 211 by means of the seal ring 241. The first end of the transmission rod 240 extends into the first accommodating cavity 221 and is connected to the brake member 230. As an example, the connecting portion 232 of the brake member 230 may be provided with a connecting hole, and the connecting hole may be sleeved on the first end of the transmission rod 240. The second end of the transmission rod 240 is connected to the first driving mechanism 260 such that the transmission rod 240 slides relative to the cylindrical body 210 under the driving of the first driving mechanism 260.

The syringe fixing device 20 may further be provided with the elastic compression member 250, and the elastic compression member 250 may be located in the first accommodating cavity 221 and the cylindrical body 210 that are in communication with each other. The first end of the elastic compression member 250 abuts against the limiting portion 201, and the second end of the elastic compression member 250 abuts against the brake member 230. When the first driving mechanism 260 drives the transmission rod 240 and the brake member 230 to slide toward the pump body 10 relative to the cylindrical body 210, the brake member 230 compresses the elastic compression member 250, and the elastic compression member 250 is deformed to exert a restoring force on the cylindrical body 210 by means of the limiting portion 201, such that the cylindrical body 210 drives the fixing block 220 to slide relative to the pump body 10, causing the fixing block 220 to abut against the barrel 410.

As an example, the elastic compression member 250 may be a compression spring. The compression spring is sleeved on the transmission rod 240, a first end of the compression spring abuts against the limiting portion 201, and a second end of the compression spring abuts against the brake member 230. It should be understood that the elastic compression member 250 may alternatively be annular rubber, or other elastic members that can be compressed and provide a restoring force, which will not be described in details in the first embodiment of the present application.

The first driving mechanism 260 may be located in the pump body 10. For example, a driving motor 261 may be mounted in the third accommodating cavity 112, and the second end of the transmission rod 240 is connected to the first driving mechanism 260. As an example, the transmission rod 240 is provided with a transmission thread on the outer side of the transmission rod close to the second end thereof. The first driving mechanism 260 may include the driving motor 261 and a transmission nut 262, the driving motor 261 is mounted on the pump body 10, an output shaft of the driving motor 261 is connected to the transmission nut 262 so as to drive the transmission nut 262 to rotate, and the transmission nut 262 is sleeved on the transmission thread. When the output shaft of the driving motor 261 rotates, the output shaft of the driving motor 261 drives the transmission nut 262 to rotate. Since the transmission rod 240 is slidably connected to the cylindrical body 210, the transmission nut 262 drives the transmission rod 240 to slide relative to the cylindrical body 210 by means of an external thread.

As an example, the first driving mechanism 260 may further include a transmission assembly. The transmission assembly is connected to the driving motor 261 and the transmission nut 262. A transmission ratio between the driving motor 261 and the transmission nut 262 may be adjusted by means of the transmission assembly. In addition, the relative positions of the transmission nut 262 and the driving motor 261 may also be adjusted by providing the transmission assembly, so that the structure of the first driving mechanism 260 is optimized, facilitating the miniaturization of the syringe fixing device 20.

As an example, the transmission nut 262 may be connected to the pump body 10 in such a way that the transmission nut is rotatable about the axis of the transmission nut 262. For example, a mounting seat 263 may be connected to the pump body 10, and the transmission nut 262 may be rotatably connected to the mounting seat 263. Transmission teeth 264 may be provided on an outer side of the transmission nut 262. The transmission assembly may include at least one transmission gear 265. The transmission gear 265 is sleeved on the output shaft of the driving motor 261 and meshes with the transmission teeth 264. When the output shaft of the driving motor 261 rotates, the output shaft of the driving motor 261 can drive at least one transmission gear 265 to rotate, and the at least one transmission gear 265 drives the transmission nut 262 to rotate by means of the transmission teeth 264.

In some possible implementations of the first embodiment of the present application, the transmission assembly may further include a timing pulley and a timing belt. The timing pulley is sleeved on the output shaft of the driving motor 261. The timing belt surrounds the timing pulley and the transmission nut 262, and the timing belt abuts against an outer surface of the timing pulley and abuts against an outer surface of the transmission nut 262. When the output shaft of the driving motor 261 rotates, the output shaft of the driving motor 261 drives the timing pulley to rotate, and the timing pulley drives the transmission nut 262 to rotate by means of the timing belt.

It should be understood that the transmission assembly may alternatively be transmission mechanisms in other forms, which will not be described in details in the first embodiment of the present application.

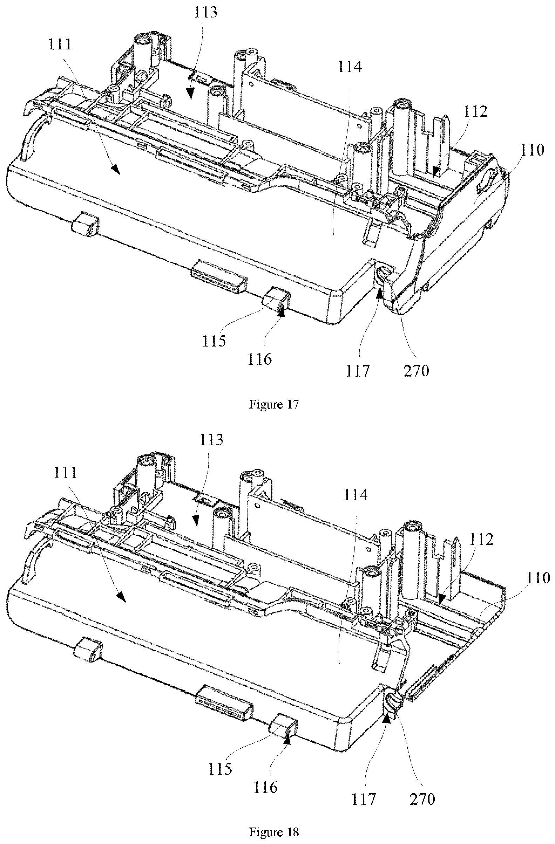

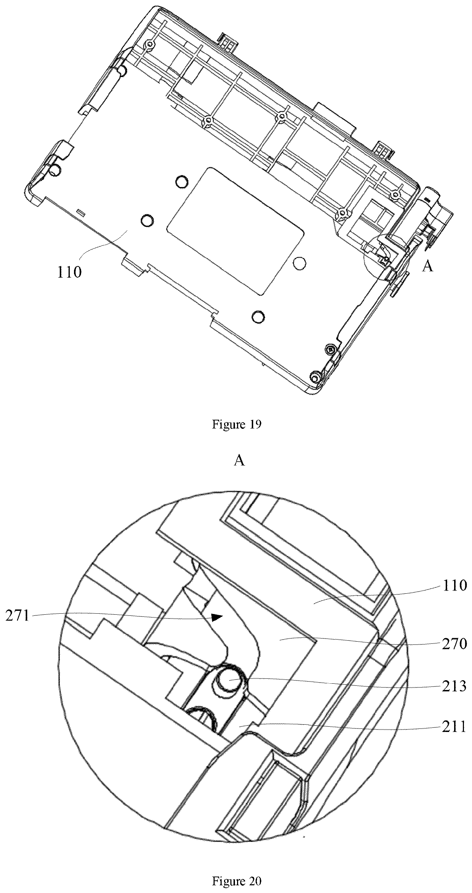

The cylindrical body 210 can slide relative to the pump body 10. As an example, referring to FIGS. 17, 18, 19 and 20, the syringe fixing device 20 may further include a guide ring 270. The guide ring 270 is mounted in the connecting through hole 117 of the pump body 10. As an example, the guide ring 270 and the first housing 110 may be of an integral structure. A guide groove 271 is provided on an inner side of the guide ring 270. The cylindrical body 210 is inserted into the guide ring 270, a guide protrusion 213 is provided on the outer side of the cylindrical body 210 close to the second end thereof, and the guide protrusion 213 is slidably arranged in the guide groove 271. When the first driving mechanism 260 drives the cylindrical body 210 to slide relative to the pump body 10 by means of the transmission rod 240 and the brake member 230, the guide groove 271 can constrain and guide the guide protrusion 213, thereby constraining and guiding a sliding direction of the cylindrical body 210.

In some possible implementations of the first embodiment of the present application, the guide groove 271 may also be a linear groove parallel to the axis of the guide ring 270. When the first driving mechanism 260 acts, the first driving mechanism 260 drives the transmission rod 240 to slide relative to the cylindrical body 210 in a direction away from the pump body 10, the transmission rod 240 drives the brake member 230 to slide in the direction away from the pump body 10, and the brake member 230 pushes the fixing block 220, the cylindrical body 210 and the guide protrusion 213 to slide along the linear groove in the direction away from the pump body 10, so that the fixing block 220 is moved away from the first accommodating cavity 221, the syringe 40 is prevented from interfering with the fixing block 220 when the syringe 40 is placed in the first accommodating cavity 221, and the convenience of placing the syringe 40 is improved.

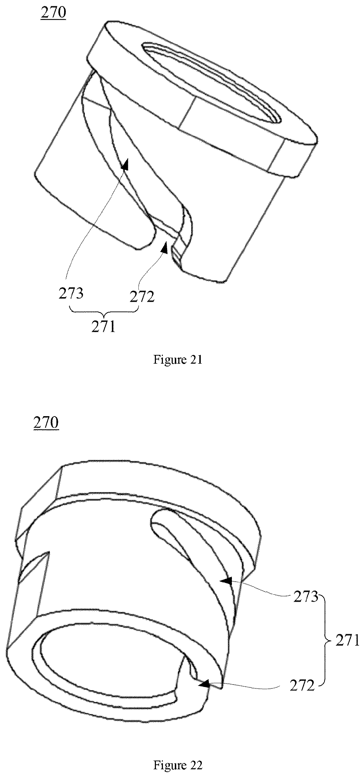

In other possible implementations of the first embodiment of the present application, referring to FIGS. 21 and 22, the guide groove 271 may include a linear groove section 272 and a spiral groove section 273 which are connected to each other. An extension direction of the linear groove section 272 is parallel to the axis of the guide ring 270, and the linear groove section 272 has a guide inlet formed at the second end of the cylindrical body 210. The spiral groove section 273 is closer to the fixing block 220 than the linear groove section 272, and an extension direction of the spiral groove section 273 surrounds the axis of the guide ring 270.

When the first driving mechanism 260 acts, the first driving mechanism 260 drives the transmission rod 240 to slide relative to the cylindrical body 210 in the direction away from the pump body 10, the transmission rod 240 drives the brake member 230 to slide in the direction away from the pump body 10, the brake member 230 pushes the fixing block 220 and the cylindrical body 210 to slide relative to the pump body 10 in the direction away from the pump body 10, the guide protrusion 213 enters the linear groove section 272 through the guide inlet, and the linear groove section 272 functions to guide the guide protrusion 213, such that the cylindrical body 210 and the fixing block 220 are moved away from the pump body 10 in the extension direction of the linear groove section 272. When the syringe 40 is placed in the first accommodating cavity 221, the fixing block 220 can move away from the syringe 40 in the extension direction perpendicular to the syringe 40 so as to be spaced apart from the syringe 40, thereby avoiding the phenomenon that the position of the syringe 40 changes due to the friction occurring between the fixing block 220 and the syringe 40.

The guide protrusion 213 enters the spiral groove section 273 from the linear groove section 272, and the spiral groove section 273 functions to guide the guide protrusion 213, such that the cylindrical body 210 and the fixing block 220 are moved away from the pump body 10 in the extension direction of the spiral groove section 273. That is, the cylindrical body 210 and the fixing block 220 are rotated outwardly while moving away from the pump body 10, so that the blocking of the fixing block 220 to the first accommodating cavity 221 is further reduced or eliminated, and the convenience of placing and removing the syringe 40 is further improved.

When the first driving mechanism 260 acts reversely, the first driving mechanism 260 drives the transmission rod 240 to slide toward the pump body 10 relative to the cylindrical body 210, the transmission rod 240 drives the brake member 230 to slide toward the pump body 10, the brake member 230 pushes the elastic compression member 250 and compresses the elastic compression member 250, and the elastic compression member 250 exerts a restoring force on the limiting portion 201. The spiral groove section 273 functions to guide the guide protrusion 213, such that the cylindrical body 210 and the fixing block 220 are moved close to the pump body 10 in the extension direction of the spiral groove section 273. That is, the cylindrical body 210 and the fixing block 220 are rotated inwardly while moving close to the pump body 10.

The guide protrusion 213 enters the linear groove section 272 from the spiral groove section 273. The linear groove section 272 functions to guide the guide protrusion 213, such that the cylindrical body 210 and the fixing block 220 are moved close to the pump body 10 in the extension direction of the linear groove section 272. When the syringe 40 is placed in the first accommodating cavity 221, the fixing block 220 can move close to the syringe 40 in the extension direction perpendicular to the syringe 40 until the fixing block abuts against the syringe 40, thereby avoiding the phenomenon that the position of the syringe 40 changes due to the friction occurring between the fixing block 220 and the syringe 40.

It should be noted that the outward rotation of the cylindrical body 210 and the fixing block 220 refers to the rotation of the cylindrical body 210 and the fixing block 220 in direction m shown in FIG. 3. The inward rotation of the cylindrical body 210 and the fixing block 220 refers to the rotation of the cylindrical body 210 and the fixing block 220 in direction n shown in FIG. 4.

In some possible implementations of the first embodiment of the present application, the guide protrusion 213 and the cylindrical body 210 may be of an integral structure, and the guide protrusion 213 may be machined directly on the outer side of the cylindrical body 210.

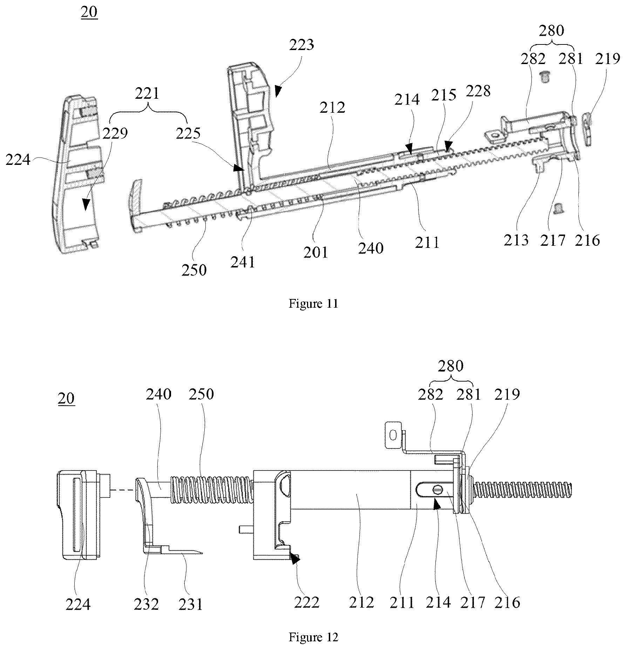

In other possible implementations of the first embodiment of the present application, the guide protrusion 213 and the cylindrical body 210 may alternatively be of a split structure. As an example, referring to FIGS. 10 and 11, a mounting groove 214 is provided in a side surface of the cylindrical body 210 close to the second end thereof, and the mounting groove 214 has a mounting opening formed at the second end of the cylindrical body 210. The second end of the cylindrical body 210 is provided with a connecting cylinder 215, and the connecting cylinder 215 has a smaller radius than the cylindrical body 210. The syringe fixing device 20 may further include a first mounting frame. The first mounting frame may include a first mounting ring 216 and a first connecting arm 217, and the first mounting ring 216 is sleeved on the connecting cylinder 215. The first connecting arm 217 is arranged in an axial direction of the first mounting ring 216 and is connected to the first mounting ring 216, and the first connecting arm 217 is mounted in the mounting groove 214. The guide protrusion 213 is provided on a side of the first connecting arm 217 facing away from the mounting groove 214.

In the process of mounting and adjusting the syringe fixing device 20, when it is necessary to adjust the shape, size, position and other parameters of the guide protrusion 213, the first mounting frame can be removed from the cylindrical body 210, the guide protrusion 213 can then be adjusted, and the first mounting frame is mounted on the cylindrical body 210 after adjustment, without the need for removing and mounting the cylindrical body 210, so that the convenience of adjusting the guide protrusion 213 is improved.

The syringe fixing device 20 may further include a size sensor. The size sensor is connected to the cylindrical body 210 and is close to the second end of the cylindrical body 210, and the size sensor is configured to identify the size of the syringe 40. For example, the size sensor may be a sliding potentiometer, or other types of sensors capable of identifying the size of the syringe 40, which will not be described in details in the first embodiment of the present application.

As an example, referring to FIGS. 10 and 11, the syringe fixing device 20 may further include a second mounting frame 280. The second mounting frame 280 may include a second mounting ring 281 and a second connecting arm 282, and the second mounting ring 281 is sleeved on the connecting cylinder 215 and abuts against the first mounting ring 216. The second connecting arm 282 is arranged in the axial direction of the second mounting ring 281, a first end of the second connecting arm 282 is connected to the second mounting ring 281, and a second end of the second connecting arm 282 is configured to mount the size sensor.

In the process of mounting and adjusting the syringe fixing device 20, when it is necessary to adjust parameters of the size sensor such as the position, the second mounting frame can be removed from the cylindrical body 210, the second mounting frame and/or the size sensor can be adjusted, the second mounting frame is then mounted on the cylindrical body 210 after adjustment, without the need for removing and mounting the cylindrical body 210, so that the convenience of adjusting the size sensor is improved.

As an example, an engagement groove 218 may be provided on an outer side of the connecting cylinder 215, and the engagement groove 218 may be located on a side of the second mounting ring 281 facing away from the first mounting ring 216. The syringe fixing device 20 may further include a circlip 219, and the circlip 219 is engaged in the engagement groove 218. The second mounting frame 280 and the first mounting frame are both fixed on the cylindrical body 210 by means of the circlip 219, so that it is more convenient and faster to fix and mount the second mounting frame 280 and the first mounting frame.

As an example, a wave washer 283 may be provided between the first mounting ring 216 and the second mounting ring 281, and the wave washer 283 is sleeved on the connecting cylinder 215 and abuts against each of the first mounting ring 216 and the second mounting ring 281. The wave washer 283 can exert a tensioning force on the first mounting ring 216 and the second mounting ring 281, such that the first mounting ring 216 abuts against the second end of the cylindrical body 210, and the second mounting ring 281 abuts against the circlip 219, improving the stability of the first mounting frame and the second mounting frame 280, and thus improving the positional stability of the guide protrusion 213 and the size sensor. It should be understood that the wave washer 283 may also be replaced with other elastic washers.

Referring to FIGS. 3 to 6, the syringe fixing device 20 may further include a clip 290. The clip 290 is connected to the pump body 10 and is opposite to the fixing block 220. The clip 290 can fix the flange of the barrel 410, so that the stability of the syringe 40 is improved.

As an example, the clip 290 may be linked with the cylindrical body 210, and when the cylindrical body 210 drives the fixing block 220 to fix the syringe 40, the clip 290 fixes the flange of the barrel 410. When the cylindrical body 210 drives the fixing block 220 to move away from the syringe 40, the clip 290 is moved away from the flange of the barrel 410.

As an example, the in-place sensor may be mounted on the clip 290, which will not be described in details in the first embodiment of the present application.

The syringe pump further includes a pushing device 30. The pushing device 30 is connected to the pump body 10, and the pushing device 30 is configured to be engaged with the handle 420 of the syringe 40 so as to drive the handle 420 to slide relative to the barrel 410 of the syringe 40.

As an example, referring to FIGS. 1 to 6, the pushing device 30 may include a push rod 310, a pushing portion 320 and a second driving mechanism 330. The push rod 310 can slide relative to the pump body 10 in the extension direction of the syringe 40. For example, a side wall of the first housing 110 may be provided with a sliding through hole 118, and the push rod 310 runs through the sliding through hole 118 and can slide in the sliding through hole 118. The pushing portion 320 is located on an outer side of the pump body 10, and the pushing portion 320 is connected to an end of the push rod 310 located outside the pump body 10. The second driving mechanism 330 is located in the pump body 10. For example, the second driving mechanism 330 may be mounted in the fourth accommodating cavity 113. The second driving mechanism 330 is connected to an end of the push rod 310 located inside the pump body 10.

When the second driving mechanism 330 acts, the push rod 310 and the pushing portion 320 can be driven to slide relative to the pump body 10 toward the side away from the pump body 10, such that the syringe 40 is placed in the pump body 10. When the second driving mechanism 330 acts reversely, the push rod 310 and the pushing portion 320 can be driven to slide relative to the pump body 10 toward a side close to the pump body 10, such that the pushing portion 320 pushes the handle 420 of the syringe 40 to slide toward the inside of the barrel 410 so as to inject the liquid drug in the barrel 410 into the body.

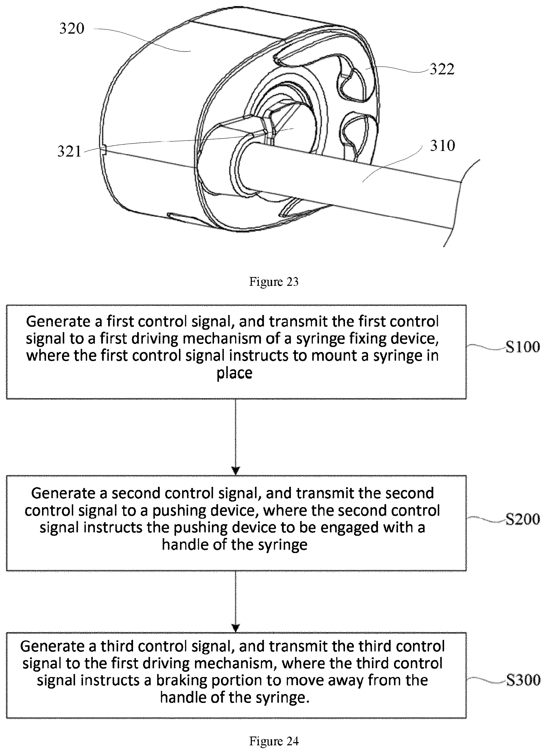

As an example, referring to FIG. 23, a pressure sensor 321 may be provided on a side of the pushing portion 320 facing the push rod 310. When the syringe 40 is placed in the pump body 10, the pushing portion 320 moves close to the handle 420 of the syringe 40, and the pressure sensor 321 is in contact with an end portion of the handle 420. The pressure sensor 321 can measure a pressure value between the pushing portion 320 and the end portion of the handle 420, and the state of the handle 420 can be determined according to the pressure value.

As an example, the pushing portion 320 may further be provided with a claw 322 on a side facing the push rod 310, and the claw 322 is configured to be engaged with the handle 420 of the syringe 40.

Referring to FIG. 24, a second embodiment of the present application provides a method for controlling a syringe pump. This method is applied to the syringe pump and may include the following steps.

In step S100, a first control signal is generated and transmitted to the first driving mechanism of the syringe fixing device, and the first control signal instructs to mount a syringe in place.

The first control signal may be configured to instruct the first driving mechanism to drive the fixing block of the syringe fixing device to abut against a barrel of the syringe, and to drive the braking portion of the brake member of the syringe fixing device to extend from the opening to abut against the handle of the syringe.

As an example, before the first control signal is generated, the pushing device 30 may first be brought into an open state. For example, the method may include a step of:
starting the syringe pump, and generating a first opening signal. The first opening signal is configured to control the pushing portion 320 of the pushing device 30 to move away from the pump body 10, and the pump body 10 is configured to mount the syringe 40. Illustratively, the syringe pump may include a controller. When the syringe pump is powered on and started, and after the controller receives a power-on signal, a first opening signal can be generated and sent to the second driving mechanism 330 of the pushing device 30. In response to the first opening signal, the second driving mechanism 330 drives the handle 310 to move the pushing portion 320 away from the pump body 10, thereby preventing the pushing portion 320 from blocking the second accommodating cavity 111 of the pump body 10, and facilitating the placement of the syringe 40 in the second accommodating cavity 111.

Alternatively, the syringe pump is started, a second operation on a second target key is received, and a first opening signal is generated in response to the second operation. As an example, after the syringe pump is powered on and started, an operator may perform the second operation on the second target key. The second target key may be the first key 121 or the second key 132, or may be a virtual key displayed on the display screen 131, etc. The second operation refers to a pressing or rotating operation and the like performed by the operator on the second target key. After receiving the second operation performed by the operator, the controller generates the first opening signal in response to the second operation, and sends the first opening signal to the second driving mechanism 330 of the pushing device 30. In response to the first opening signal, the second driving mechanism 330 drives the handle 310 to move the pushing portion 320 away from the pump body 10.

As an example, before the first control signal is generated, the door body 130 may also be brought into the open state. For example, when the syringe pump is started, the door body 130 is opened. It is possible to open the door body 130 manually after the syringe pump is powered on. A third driving mechanism may also be connected to the door body 130, and after the syringe pump is powered on and the controller receives the power-on signal, a second opening signal may be generated and sent to the third driving mechanism, and the third driving mechanism drives the door body 130 to open in response to the second opening signal.

As an example, before the first control signal is generated, the syringe fixing device 20 may also brought into in the open state. For example, when the syringe pump is powered on and started and after the controller receives the power-on signal, a third opening signal may be generated and sent to the first driving mechanism 260 of the syringe fixing device 20, the first driving mechanism 260 drives, in response to the third opening signal, the transmission rod 240 to slide relative to the cylindrical body 210 toward the side away from the pump body 10, and the transmission rod 240 drives the brake member 230 to slide relative to the cylindrical body 210 toward the side away from the pump body 10. The brake member 230 abuts against the fixing block 220 to exert a thrust on the fixing block 220, such that the fixing block 220 and the cylindrical body 210 connected to the fixing block 220 slide relative to the pump body 10 toward the side away from the pump body 10, so as to move the fixing block 220 away from the first accommodating cavity 221 to facilitate the placement of the syringe 40.

When the syringe 40 is put in place, the first control signal is generated. As an example, according to the method of the second embodiment of the present application, a first operation on a first target key may be received, and the first control signal may be generated in response to the first operation. For example, when the syringe 40 is put in place, the operator can determine a placement state of the syringe 40. If the operator confirms that the syringe 40 is put in place, the operator performs the first operation on the first target key. The first target key may be the first key 121 or the second key 132, or may be a virtual key displayed on the display screen 131, etc. The first operation refers to a pressing or rotating operation and the like performed by the operator on the first target key. As an example, after receiving the first operation performed by the operator, the controller generates the first control signal in response to the first operation.

Alternatively, it is also possible to receive an in-place signal generated by the in-place sensor, and to generate the first control signal according to the in-place signal. The in-place signal is generated by the in-place sensor when the syringe is mounted in place. For example, when the syringe 40 is put in place, the syringe 40 triggers the in-place sensor, and the in-place sensor generates the in-place signal. After receiving the in-place signal, the controller generates the first control signal according to the in-place signal.

The controller may send the first control signal to the first driving mechanism 260 of the syringe fixing device 20, and the first driving mechanism 260 brings, in response to the first control signal, the syringe fixing device 20 into the first fixing state and then into the second fixing state. That is, the first driving mechanism 260 drives the transmission rod 240 to slide the brake member 230 toward the syringe 40, the brake member 230 compresses the elastic compression member 250, the elastic compression member 250 pushes, by means of the limiting portion 201, the cylindrical body 210 and the fixing block 220 to slide toward the syringe 40, and the fixing block 220 abuts against the barrel 410 of the syringe 40 so as to fix the barrel 410. The first driving mechanism 260 continues to drive the transmission rod 240 to slide the brake member 230 toward the syringe 40, and the braking portion 231 of the brake member 230 extends from the opening 222 to abut against the handle 420 of the syringe 40 so as to fix the handle 420.

In step S200, a second control signal is generated and transmitted to the pushing device, and the second control signal instructs the pushing device to be engaged with the handle of the syringe.

As an example, after it is determined that the fixing block abuts against the barrel of the syringe and the braking portion abuts against the handle of the syringe, the second control signal may be generated.

As an example, after the fixing block 220 abuts against the barrel of the syringe 40 and the braking portion 231 abuts against the handle 420 of the syringe 40, the controller may generate the second control signal and transmit the second control signal to the second driving mechanism 330 of the pushing device 30. In response to the second control signal, the second driving mechanism 330 drives the push rod 310 to slide the pushing portion 320 toward the pump body 10 so as to be engaged with the handle 420 of the syringe 40.

As an example, it is possible to generate the second control signal after a first preset duration from the generation of the first control signal. Within the first preset duration, the syringe fixing device 20 may be switched into the first fixing state and then into the second fixing state. After the syringe fixing device 20 is switched into the second fixing state, the controller generates the second control signal to control the pushing device 30 to be engaged with the handle 420 of the syringe 40.

As an example, the operator may also determine whether the syringe fixing device 20 is in the second fixing state. If the operator confirms that the syringe fixing device 20 is in the second fixing state, a third operation is performed on a third target key. The third target key may be the first key 121 or the second key 132, or may be a virtual key displayed on the display screen 131, etc. The third operation refers to a pressing or rotating operation and the like performed by the operator on the third target key. After receiving the third operation performed by the operator, the controller may generate the second control signal in response to the third operation.

The second control signal is configured to instruct the pushing device to be engaged with the handle of the syringe. As an example, the pushing portion 320 of the pushing device 30 is provided with the pressure sensor 321 and the claw 322. When the pushing portion 320 of the pushing device 30 abuts against the handle 420 of the syringe 40, the pressure sensor 321 detects the pressure value between the pushing portion 320 and the handle 420. After obtaining the pressure value, the controller determines whether the pressure value is within a preset range. If the pressure value is within the preset range, it is determined that the pushing portion 320 is in close contact with the handle 420. The controller generates an engagement signal and sends the engagement signal to the claw 322. The claw 322 is engaged with the handle 420 in response to the engagement signal.

In step S300, a third control signal is generated and transmitted to the first driving mechanism, and the third control signal instructs the braking portion to move away from the handle of the syringe.

After the pushing portion 320 of the pushing device 30 is engaged with the handle 420 of the syringe 40, the third control signal may be generated. As an example, it is possible to generate the second control signal after a second preset duration from the generation of the engagement signal. Within the second preset duration, the claw 322 can complete the engagement of the handle 420. After the claw 322 complete the engagement of the handle 420, the controller generates the third control signal.

As an example, the claw 322 may be provided with an engagement switch. When the claw 322 is engaged with the handle 420, the engagement switch is triggered, and the engagement switch generates an engagement completion signal. After receiving the engagement completion signal, the controller generates the third control signal.

After generating the third control signal, the controller sends the third control signal to the first driving mechanism 260, the first driving mechanism 260 drives, in response to the third control signal, the braking portion 231 of the brake member 230 to retract from the opening 222, so as to move the braking portion 231 away from the handle 420 of the syringe 40, that is, to bring the syringe fixing device 20 into the first fixing state.

Next, the pushing device 30 can push the handle 420 to inject the liquid drug.

With the method for controlling a syringe pump provided in the second embodiment of the present application, the first driving mechanism can be controlled to act after the syringe is placed in the pump body, such that the syringe fixing device is in the first state, that is, the fixing block of the syringe fixing device abuts against the barrel of the syringe, so as to fix the barrel of the syringe. The first driving mechanism continues to be controlled to act such that the syringe fixing device is in the second state, that is, the braking portion of the brake pad of the syringe fixing device abuts against the handle of the syringe to fix the handle of the syringe. Accordingly, in the process of fixing the syringe, in the subsequent process of engaging the handle of the syringe, when the handle of the syringe is touched accidentally, or in other cases, the handle of the syringe can be prevented from moving relative to the barrel, and the liquid drug in the syringe can then be prevented from being injected into the body uncontrollably, so that the liquid drug injection accuracy and the safety performance of the syringe pump are improved.

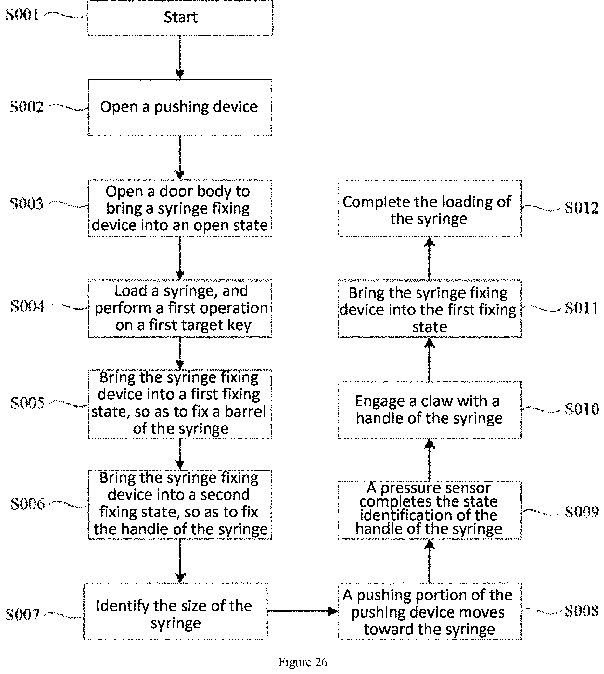

Referring to FIG. 26, a third embodiment of the present application provides a method for controlling a syringe pump. The method includes the following steps.

In step S001, start is performed.

As an example, the syringe pump may be started first by powering on the syringe pump.

In step S002, the pushing device is opened.

As an example, after receiving the power-on signal, the controller may generate the first opening signal and send the first opening signal to the second driving mechanism 330 of the pushing device 30. In response to the first opening signal, the second driving mechanism 330 drives the handle 310 to move the pushing portion 320 away from the pump body 10 so as to open the pushing device 30.

In step S003, the door body is opened to bring the syringe fixing device into the open state.

As an example, after the controller receives the power-on signal, the second opening signal may be generated and sent to the third driving mechanism, and the third driving mechanism drives the door body 130 to be opened in response to the second opening signal. Alternatively, the operator may open the door body 130 manually.

As an example, when the syringe pump is powered on and started and after the controller receives the power-on signal, the third opening signal may be generated and sent to the first driving mechanism 260 of the syringe fixing device 20, the first driving mechanism 260 drives, in response to the third opening signal, the transmission rod 240 to slide relative to the cylindrical body 210 toward the side away from the pump body 10, and the transmission rod 240 drives the brake member 230 to slide relative to the cylindrical body 210 toward the side away from the pump body 10. The brake member 230 abuts against the fixing block 220 to exert a thrust on the fixing block 220, such that the fixing block 220 and the cylindrical body 210 connected to the fixing block 220 slide relative to the pump body 10 toward the side away from the pump body 10, to bring the syringe fixing device 20 into the open state.

Alternatively, when the door body 130 is opened, the door state sensor is triggered, and the door state sensor emits the door opening signal. After receiving the door opening signal, the controller generates the third opening signal and sends the third opening signal to the first driving mechanism 260 of the syringe fixing device 20, and the first driving mechanism 260 brings, in response to the third opening signal, the syringe fixing device 20 into the open state.

In step S004, the syringe is loaded, and the in-place sensor identifies whether the syringe is put in place.

As an example, the syringe 40 is placed in the second accommodating cavity 111 of the pump body 10. The syringe 40 triggers the in-place sensor, and the in-place sensor generates the in-place signal. After receiving the in-place signal, the controller generates the first control signal according to the in-place signal.

In step S005, the syringe fixing device is brought into the first fixing state, so as to fix the barrel of the syringe.

As an example, the controller sends the first control signal to the first driving mechanism 260 of the syringe fixing device 20. The first driving mechanism 260 drives, in response to the first control signal, the transmission rod 240 to slide the brake member 230 toward the syringe 40, the brake member 230 compresses the elastic compression member 250, the elastic compression member 250 pushes, by means of the limiting portion 201, the cylindrical body 210 and the fixing block 220 to slide toward the syringe 40, and the fixing block 220 abuts against the barrel 410 of the syringe 40 so as to fix the barrel 410.

In step S006, the syringe fixing device is brought into the second fixing state, so as to fix the handle of the syringe.

As an example, in response to the first control signal, the first driving mechanism 260 continues to drive the transmission rod 240 to slide the brake member 230 toward the syringe 40, and the braking portion 231 of the brake member 230 extends from the opening 222 to abut against the handle 420 of the syringe 40 so as to fix the handle 420.

As an example, in the process of bringing the syringe fixing device 20 into the first fixing state and then into the second fixing state, the controller may further generate a fixing signal and send the fixing signal to the clip 290, and in response to the fixing signal, the clip 290 abuts against the barrel 410 so as to fix the barrel 410.

Optionally, the clip 290 may also be configured to be mechanically linked with the syringe fixing device 20. In the process of switching the syringe fixing device 20 into the first fixing state, the clip 290 may be driven to fix the barrel 410.

In step S007, the size of the syringe is identified.

As an example, when the first driving mechanism 260 drives the syringe fixing device 20 into the first fixing state and the second fixing state, the size sensor follows the cylindrical body 210 to move, and can identify the size of the syringe 40 and generate size information. The controller can obtain the size information.

In step S008, the pushing portion of the pushing device moves toward the syringe.

As an example, after the fixing block 220 abuts against the barrel of the syringe 40 and the braking portion 231 abuts against the handle 420 of the syringe 40, the controller may generate the second control signal and transmit the second control signal to the second driving mechanism 330 of the pushing device 30. In response to the second control signal, the second driving mechanism 330 drives the push rod 310 to slide the pushing portion 320 toward the pump body 10.

In step S009, the pressure sensor completes the state identification of the handle of the syringe.

As an example, the pushing portion 320 of the pushing device 30 is provided with the pressure sensor 321 and the claw 322. When the pushing portion 320 of the pushing device 30 abuts against the handle 420 of the syringe 40, the pressure sensor 321 detects the pressure value between the pushing portion 320 and the handle 420, and identifies the state of the push rod 420 according to the pressure value. For example, after obtaining the pressure value, the controller can determine whether the pressure value is within the preset range. If the pressure value is within the preset range, it is determined that the pushing portion 320 is in close contact with the handle 420.

In step S010, the claw is engaged with the handle of the syringe.

As an example, after determining that the pushing portion 320 is in close contact with the handle 420, the controller may generate the engagement signal and send the engagement signal to the claw 322. The claw 322 is engaged with the handle 420 in response to the engagement signal.

In step S011, the syringe fixing device is brought into the first fixing state.

After the pushing portion 320 of the pushing device 30 is engaged with the handle 420 of the syringe 40, the third control signal may be generated. After generating the third control signal, the controller sends the third control signal to the first driving mechanism 260, the first driving mechanism 260 drives, in response to the third control signal, the braking portion 231 of the brake member 230 to retract from the opening 222, so as to move the braking portion 231 away from the handle 420 of the syringe 40, that is, to bring the syringe fixing device 20 into the first fixing state.

In step S012, the loading of the syringe is completed.

After the loading of the syringe 40 is completed, the pushing device 30 can push the handle 420 to inject the liquid drug into the body.

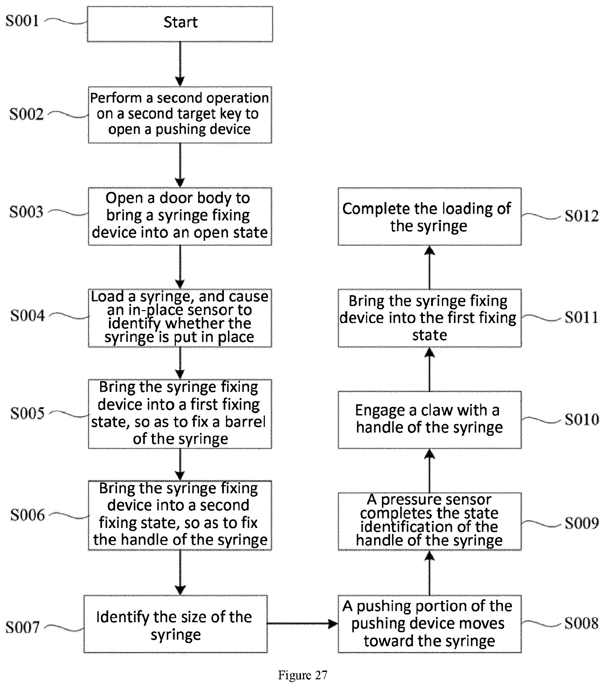

Referring to FIG. 27, a fourth embodiment of the present application provides a method for controlling a syringe pump. The difference between the fourth embodiment and the third embodiment of the present application lies in step S004 in which the syringe is loaded, and the first operation is performed on the first target key.

As an example, when the syringe 40 is put in place, the operator can determine the placement state of the syringe 40. If the operator confirms that the syringe 40 is put in place, the operator performs the first operation on the first target key. The first target key may be the first key 121 or the second key 132, or may be a virtual key displayed on the display screen 131, etc. The first operation refers to a pressing or rotating operation and the like performed by the operator on the first target key. As an example, after receiving the first operation performed by the operator, the controller generates the first control signal in response to the first operation.

For other steps in the fourth embodiment of the present application, reference may be made to the third embodiment, and these steps will not be described in details.

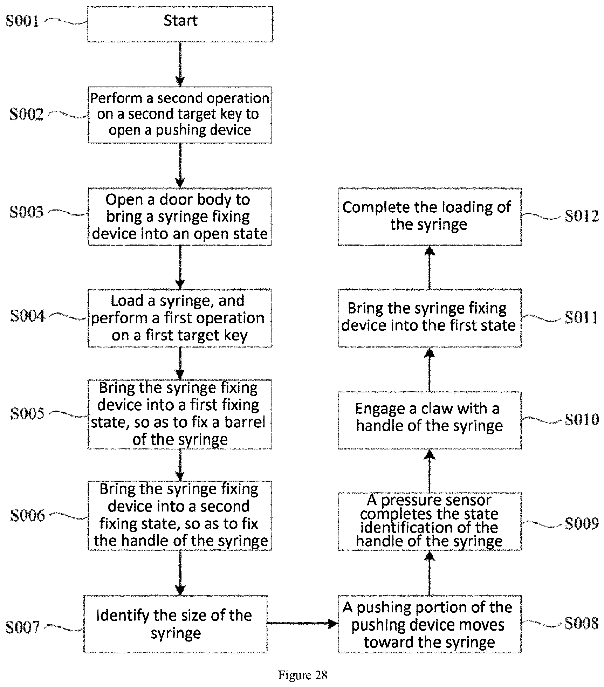

Referring to FIGS. 27 and 28, a fifth embodiment of the present application provides a method for controlling a syringe pump. The difference between the fifth embodiment and the third and fourth embodiments of the present application lies in step S002 in which the second operation is performed on the second target key to open the pushing device.

As an example, after the syringe pump is powered on and started, the operator may perform the second operation on the second target key. The second target key may be the first key 121 or the second key 132, or may be a virtual key displayed on the display screen 131, etc. The second operation refers to a pressing or rotating operation and the like performed by the operator on the second target key. After receiving the second operation performed by the operator, the controller generates the first opening signal in response to the second operation, and sends the first opening signal to the second driving mechanism 330 of the pushing device 30. In response to the first opening signal, the second driving mechanism 330 drives the push rod 310 to move the pushing portion 320 away from the pump body 10.

For other steps in the fifth embodiment of the present application, reference may be made to the third embodiment and the fourth embodiment, and these steps will not be described in details.

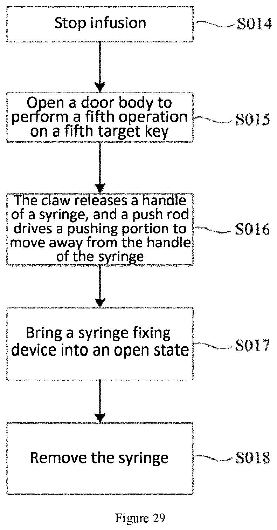

Referring to FIG. 29, a sixth embodiment of the present application provides a method for controlling a syringe pump. The control method includes the following steps.

In step S014, infusion is stopped.

As an example, the infusion may be stopped after the pushing device 30 injects the liquid drug according to a specific procedure. It is also possible that after the operator performs a fourth operation on a fourth target key, the controller generates an infusion stop signal according to the fourth operation, and sends the infusion stop signal to the pushing device 30, and the pushing device 30 stops the infusion in response to the infusion stop signal.

In step S015, the door body is opened to perform a fifth operation on a fifth target key.

As an example, when the pushing device 30 stops the infusion, the pushing device 30 may generate an infusion completion signal. After receiving the infusion completion signal, the controller generates a fourth control signal and sends the fourth control signal to the third driving mechanism of the door body 130. The fourth control signal is configured to instruct the third driving mechanism to open the door body 130.

After the door body 130 is opened, the operator can perform the fifth operation on the fifth target key. As an example, the fifth target key may be the first key 121 or the second key 132, or may be a virtual key displayed on the display screen 131, etc. The fifth operation refers to a pressing or rotating operation and the like performed by the operator on the fifth target key.

In step 5016, the claw releases the handle of the syringe, and the push rod drives the pushing portion to move away from the handle of the syringe.

As an example, after receiving the fifth operation performed by the operator, the controller generates a fifth control signal in response to the fifth operation and sends the fifth control signal to the claw 322 of the pushing device 30, and the claw 322 releases the handle 420 in response to the fifth control signal.

After the claw 322 releases the handle 420, the second driving mechanism 330 generates a sixth control signal in response to the action of the claw 322 releasing the handle 420, and sends the sixth control signal to the second driving mechanism 330 of the pushing device 30. In response to the sixth control signal, the second driving mechanism 330 drives the push rod 310 to move away from the pump body 10 such that the pushing portion 320 moves away from the handle 420 of the syringe 40.

In step S017, the syringe fixing device is brought into the open state.

After the pushing portion 320 moves away from the handle 420 of the syringe 40, the pressure sensor 321 detects that the pressure between the pushing portion 320 and the handle 420 is 0. When obtaining the pressure of 0 between the pushing portion 320 and the handle 420, the controller generates a seventh control signal and sends the seventh control signal to the first driving mechanism 260 of the syringe fixing device 20, the first driving mechanism 260 drives, in response to the seventh control signal, the transmission rod 240 to slide the brake member 230 relative to the cylindrical body 210 toward the side away from the pump body 10, the brake member 230 pushes the fixing block 220 and the cylindrical body 210 to slide toward the side away from the pump body 10, and the fixing block 220 is moved away from the barrel 410 of the syringe 40.

In step S018, the syringe is removed.

The syringe 40 is taken out from the second accommodating cavity 111 of the pump body 10.

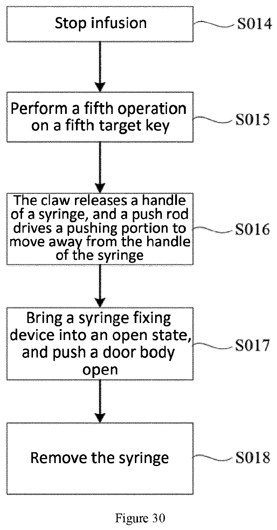

Referring to FIG. 30, a seventh embodiment of the present application provides a method for controlling a syringe pump. The differences between the seventh embodiment and the sixth embodiment of the present application lie in step S015 in which the fifth operation is performed on the fifth target key; and step S017 in which the syringe fixing device is brought into the open state, and the door body is pushed open.

As an example, when the pushing device 30 stops the infusion, the operator may perform the fifth operation on the fifth target key. As an example, the fifth target key may be the first key 121 or the second key 132, or may be a virtual key displayed on the display screen 131, etc. The fifth operation refers to a pressing or rotating operation and the like performed by the operator on the fifth target key.

After receiving the fifth operation performed by the operator, the controller generates, in response to the fifth operation, the fifth control signal and the sixth control signal, and sends the fifth control signal and the sixth signal to the pushing device 30, such that the claw 322 releases the handle 420, and the pushing portion 320 moves away from the handle 420.

After the pushing portion 320 moves away from the handle 420 of the syringe 40, the pressure sensor 321 detects that the pressure between the pushing portion 320 and the handle 420 is 0. When obtaining the pressure of 0 between the pushing portion 320 and the handle 420, the controller generates a seventh control signal and sends the seventh control signal to the first driving mechanism 260 of the syringe fixing device 20, the first driving mechanism 260 drives, in response to the seventh control signal, the transmission rod 240 to slide the brake member 230 relative to the cylindrical body 210 toward the side away from the pump body 10, the brake member 230 pushes the fixing block 220 and the cylindrical body 210 to slide toward the side away from the pump body 10, and the fixing block 220 is moved away from the barrel 410 of the syringe 40.

The fixing block 220 pushes the door body 130 open in the process of moving away from the barrel 410 of the syringe 40, so that there is no need to control the opening of the door body 130, and thus a control step is omitted.

For other steps in the seventh embodiment of the present application, reference may be made to the sixth embodiment, and these steps will not be described in details.

It should be finally noted that the above embodiments are merely used for illustrating rather than limiting the technical solution of the present application. Although the present application has been illustrated in detail with reference to the foregoing embodiments, it should be understood by those of ordinary skill in the art that the technical solutions described in the above embodiments can still be modified, or some or all of the technical features in the technical solutions can be equivalently substituted; and these modifications or substitutions do not make the essence of the corresponding technical solution depart from the scope of the technical solutions of the embodiments of the present application.

## Claims

1. A syringe fixing device (20) for fixing a syringe (40) placed in a pump body (10), the syringe fixing device (20) comprising:
a cylindrical body (210) connected to the pump body (10) and slidable relative to the pump body (10), an inner wall of the cylindrical body (210) being provided with a limiting portion (201);
a contact block connected to an end of the cylindrical body (210) and abutting against a barrel (410) of the syringe (40), a first accommodating cavity (221) in communication with the cylindrical body (210) being provided in the contact block, and the first accommodating cavity (221) having an opening (222) facing the syringe (40);
a brake member (230) located in the first accommodating cavity (221), the brake member (230) having a braking portion (231) opposite to the opening (222);
a transmission rod (240) slidably arranged in the cylindrical body (210), a first end of the transmission rod (240) extending into the first accommodating cavity (221) and being connected to the brake member (230);
an elastic compression member (250) located in the first accommodating cavity (221) and the cylindrical body (210) that are in communication with each other, a first end of the elastic compression member (250) abutting against the limiting portion (201), and a second end of the elastic compression member (250) abutting against the brake member (230); and
a first driving mechanism (260) connected to a second end of the transmission rod (240), for driving the transmission rod (240) to slide relative to the cylindrical body (210);
wherein when the syringe fixing device (20) is in a first fixing state, the first driving mechanism (260) drives the transmission rod (240) to slide the brake member (230) toward the syringe (40), the brake member (230) compresses the elastic compression member (250), the elastic compression member (250) pushes, by means of the limiting portion (201), the cylindrical body (210) and the contact block to slide toward the syringe (40), and the contact block abuts against the barrel (410) of the syringe (40); and when the syringe fixing device (20) is in a second fixing state, the first driving mechanism (260) continues to drive the transmission rod (240) to slide the brake member (230) toward the syringe (40), and the braking portion (231) of the brake member (230) extends from the opening (222) to abut against a handle (420) of the syringe (40).

2. The syringe fixing device (20) according to claim 1, wherein the contact block is a fixing block (220), and the fixing block (220) is connected to a first end of cylindrical body (210) away from the pump body (10); and/or
the contact block is a fixing block (220), and the fixing block (220) is connected to a first end of cylindrical body (210) away from the pump body (10); the transmission rod (240) is provided with a transmission thread on an outer side of the transmission rod (240) close to the second end thereof; and
the first driving mechanism (260) comprises a driving motor (261) and a transmission nut (262), the driving motor (261) is mounted on the pump body (10), an output shaft of the driving motor (261) is connected to the transmission nut (262) so as to drive the transmission nut (262) to rotate, and the transmission nut (262) is sleeved on the transmission thread; and/or
the contact block is a fixing block (220), and the fixing block (220) is connected to a first end of cylindrical body (210) away from the pump body (10); the transmission rod (240) is provided with a transmission thread on an outer side of the transmission rod (240) close to the second end thereof; and the first driving mechanism (260) comprises a driving motor (261) and a transmission nut (262), the driving motor (261) is mounted on the pump body (10), an output shaft of the driving motor (261) is connected to the transmission nut (262) so as to drive the transmission nut (262) to rotate, and the transmission nut (262) is sleeved on the transmission thread; the first driving mechanism (260) further comprises a transmission assembly, the transmission assembly being connected to the driving motor (261) and the transmission nut (262); and/or
the contact block is a fixing block (220), and the fixing block (220) is connected to a first end of cylindrical body (210) away from the pump body (10); the transmission rod (240) is provided with a transmission thread on an outer side of the transmission rod (240) close to the second end thereof; and the first driving mechanism (260) comprises a driving motor (261) and a transmission nut (262), the driving motor (261) is mounted on the pump body (10), an output shaft of the driving motor (261) is connected to the transmission nut (262) so as to drive the transmission nut (262) to rotate, and the transmission nut (262) is sleeved on the transmission thread; the first driving mechanism (260) further comprises a transmission assembly, the transmission assembly being connected to the driving motor (261) and the transmission nut (262); the transmission nut (262) is connected to the pump body (10) in such a way that the transmission nut (262) is rotatable about an axis of the transmission nut (262), and transmission teeth (264) are provided on an outer side of the transmission nut (262); and the transmission assembly comprises at least one transmission gear (265), the transmission gear (265) being sleeved on the output shaft of the driving motor (261) and meshing with the transmission teeth (264).

3. The syringe fixing device (20) according to claim 2, wherein the brake member (230) further comprises a connecting portion (232), a first end of the connecting portion (232) is connected to the first end of the transmission rod (240), a second end of the connecting portion (232) is connected to the braking portion (231), and the connecting portion (232) abuts against the fixing block (220); and/or
the brake member (230) further comprises a connecting portion (232), a first end of the connecting portion (232) is connected to the first end of the transmission rod (240), a second end of the connecting portion (232) is connected to the braking portion (231), and the connecting portion (232) abuts against the fixing block (220); the connecting portion (232) is of an arc-shaped structure which is bent toward a side away from the pump body (10).

4. The syringe fixing device (20) according to claim 2, wherein the fixing block (220) comprises a first fixing portion (223) and a second fixing portion (224) which are connected to each other, the first fixing portion (223) is connected to an end of the cylindrical body (210) facing away from the pump body (10), a first accommodating groove (225) is provided on a side of the first fixing portion (223) facing away from the cylindrical body (210), and the opening (222) is provided at a bottom of the first accommodating groove (225); and
the second fixing portion (224) is located on the side of the first fixing portion (223) facing away from the cylindrical body (210), a second accommodating groove (229) is provided on a side of the second fixing portion (224) facing the first fixing portion (223), and the second accommodating groove (229) and the first accommodating groove (225) enclose the first accommodating cavity (221).

5. The syringe fixing device (20) according to claim 4, wherein the first fixing portion (223) comprises a first fixing sub-portion (226) and a second fixing sub-portion (227) which are arranged in a radial direction of the cylindrical body (210) and connected to each other; a part of the second fixing sub-portion (227) protrudes from an end surface of the first fixing sub-portion (226) facing the pump body (10), and the first fixing sub-portion (226) and the part of the second fixing sub-portion (227) protruding from the first fixing sub-portion (226) form a press-connecting groove (228) configured to be engaged with a flange of the barrel (410); and the opening (222) is provided in the second fixing sub-portion (227) and is located on a side of the press-connecting groove (228) facing away from the pump body (10).

6. The syringe fixing device (20) according to claim 2, further comprising a guide ring (270), wherein the guide ring (270) is mounted in a connecting through hole of the pump body (10), and a guide groove (271) is provided on an inner side of the guide ring (270); and
the cylindrical body (210) is inserted into the guide ring (270), a guide protrusion (213) is provided on an outer side of the cylindrical body (210) close to a second end thereof, and the guide protrusion (213) is slidably arranged in the guide groove (271).

7. The syringe fixing device (20) according to claim 6, wherein the guide groove (271) comprises a linear groove section (272) and a spiral groove section (273) which are connected to each other, an extension direction of the linear groove section (272) is parallel to an axis of the guide ring (270), and the linear groove section (272) has a guide inlet formed at the second end of the cylindrical body (210); and the spiral groove section (273) is closer to the fixing block (220) than the linear groove section (272), and an extension direction of the spiral groove section (273) surrounds the axis of the guide ring (270).

8. The syringe fixing device (20) according to claim 6, wherein a mounting groove (214) is provided in a side surface of the cylindrical body (210) close to the second end thereof, and the mounting groove (214) has a mounting opening formed at the second end of the cylindrical body (210); the second end of the cylindrical body (210) is provided with a connecting cylinder (215), and the connecting cylinder (215) has a smaller radius than the cylindrical body (210);
the syringe fixing device (20) further comprises a first mounting frame, the first mounting frame comprises a first mounting ring (216) and a first connecting arm (217), and the first mounting ring (216) is sleeved on the connecting cylinder (215); the first connecting arm (217) is arranged in an axial direction of the first mounting ring (216) and is connected to the first mounting ring (216), and the first connecting arm (217) is mounted in the mounting groove (214); and the guide protrusion (213) is provided on a side of the first connecting arm (217) facing away from the mounting groove (214).

9. The syringe fixing device (20) according to claim 8, further comprising a size sensor, wherein the size sensor is connected to the cylindrical body (210) and is close to the second end of the cylindrical body (210), and the size sensor is configured to identify the size of the syringe (40); and/or
the size sensor is connected to the cylindrical body (210) and is close to the second end of the cylindrical body (210), and the size sensor is configured to identify the size of the syringe (40); the syringe fixing device (20) further comprises a second mounting frame (280), wherein the second mounting frame (280) comprises a second mounting ring (281) and a second connecting arm (282), and the second mounting ring (281) is sleeved on the connecting cylinder (215) and abuts against the first mounting ring (216); and
the second connecting arm (282) is arranged in an axial direction of the second mounting ring (281), a first end of the second connecting arm (282) is connected to the second mounting ring (281), and a second end of the second connecting arm (282) is configured to mount the size sensor; and/or
the size sensor is connected to the cylindrical body (210) and is close to the second end of the cylindrical body (210), and the size sensor is configured to identify the size of the syringe (40); the syringe fixing device (20) further comprises a second mounting frame (280), wherein the second mounting frame (280) comprises a second mounting ring (281) and a second connecting arm (282), and the second mounting ring (281) is sleeved on the connecting cylinder (215) and abuts against the first mounting ring (216); and the second connecting arm (282) is arranged in an axial direction of the second mounting ring (281), a first end of the second connecting arm (282) is connected to the second mounting ring (281), and a second end of the second connecting arm (282) is configured to mount the size sensor; an engagement groove (218) is provided on an outer side of the connecting cylinder (215), and the engagement groove (218) is located on a side of the second mounting ring (281) facing away from the first mounting ring (216); and
the syringe fixing device (20) further comprises a circlip (219) engaged in the engagement groove (218); and/or
the size sensor is connected to the cylindrical body (210) and is close to the second end of the cylindrical body (210), and the size sensor is configured to identify the size of the syringe (40); the syringe fixing device (20) further comprises a second mounting frame (280), wherein the second mounting frame (280) comprises a second mounting ring (281) and a second connecting arm (282), and the second mounting ring (281) is sleeved on the connecting cylinder (215) and abuts against the first mounting ring (216); and the second connecting arm (282) is arranged in an axial direction of the second mounting ring (281), a first end of the second connecting arm (282) is connected to the second mounting ring (281), and a second end of the second connecting arm (282) is configured to mount the size sensor; an engagement groove (218) is provided on an outer side of the connecting cylinder (215), and the engagement groove (218) is located on a side of the second mounting ring (281) facing away from the first mounting ring (216); and the syringe fixing device (20) further comprises a circlip (219) engaged in the engagement groove (218); a wave washer (219) is provided between the first mounting ring (216) and the second mounting ring (281), and the wave washer (219) is sleeved on the connecting cylinder (215) and abuts against each of the first mounting ring (216) and the second mounting ring (281).

10. The syringe fixing device (20) according to claims 2, wherein the cylindrical body (210) comprises a first cylindrical body (211) and a second cylindrical body (212), and the first cylindrical body (211) is slidable relative to the pump body (10);

11. a first end of the second cylindrical body (212) is sleeved on a first end of the first cylindrical body (211) away from the pump body (10), and is fixedly connected to the first cylindrical body (211) for the transmission rod (240) to pass through; and the first end of the first cylindrical body (211) is the limiting portion (201), and a second end of the second cylindrical body (212) is connected to the fixing block (220).

12. A method for controlling a syringe pump, comprising:
generating a first control signal, and transmitting the first control signal to a first driving mechanism of a syringe fixing device, wherein the first control signal instructs to mount a syringe in place;
generating a second control signal, and transmitting the second control signal to a pushing device, wherein the second control signal instructs the pushing device to be engaged with a handle of the syringe; and
generating a third control signal, and transmitting the third control signal to the first driving mechanism, wherein the third control signal instructs the braking portion to move away from a handle of the syringe.

13. The method according to claim 12, wherein the method comprises:
generating a first control signal upon detecting a syringe being placed in place; and transmitting the first control signal to the first drive mechanism of the syringe fixing device; wherein the first control signal instructs the first drive mechanism to drive a contact block of the syringe fixing device to abut the barrel of the syringe and to drive the braking portion of a braking member of the syringe fixing device to extend from an opening to abut a barrel of the syringe;
generating a second control signal and transmitting the second control signal to the pushing device after determining that the contact block is abutting the barrel of the syringe and the braking portion is abutting the handle of the syringe; wherein the second control signal instructs the pushing device is seated with the handle of the syringe;
generating a third control signal and transmitting the third control signal to the first driving mechanism; wherein the third control signal instructs the first driving mechanism to drive the braking portion of the braking member to retract from the opening so as to disengage the braking portion from the handle of the syringe.

14. The method according to claim 12 or 13, wherein the method is applied to the syringe pump according to claim 11.

15. The method according to claim 12, wherein the syringe pump further comprises an in-place sensor; and the step of generating the first control signal comprises:
receiving a first operation on a first target key; and generating the first control signal in response to the first operation;
or receiving an in-place signal generated by the in-place sensor, and generating the first control signal according to the in-place signal; and/or
the pushing device comprises a pushing portion engaged with the syringe; and
before the step of generating the first control signal, the method further comprises:
starting the syringe pump, and generating a first opening signal;
or starting the syringe pump, receiving a second operation on a second target key, and generating a first opening signal in response to the second operation; wherein the first opening signal controls the pushing portion to move away from the pump body.
